# EUROPEAN PATENT SPECIFICATION

(11) **EP 0 714 417 B1**
(45) Date of publication and mention of the grant of the patent: **10.06.1998**
(21) Application number: 94923807.5
(22) Date of filing: 17.08.1994
(51) Int. Cl.: C08F 246/00, C07F 9/09, C09D 157/12

(54) **POLYMER SURFACE COATINGS**
POLYMEROBERFLÄCHENBESCHICHTUNGSZUSAMMENSETZUNG
PROCEDES DE REVETEMENT DE SURFACE AVEC DES POLYMERES

(30) Priority: 18.08.1993 GB 9317178
(43) Date of publication of application: 05.06.1996
(73) Proprietor: BIOCOMPATIBLES LIMITED, Farnham, Surrey GU9 8QL (GB)
(72) Inventor: NAKABAYASHI, Nobuo, Inst. Medical & Dental Eng.,, Kanda, Tokyo 101 (JP); ISHIHARA, Kazuhiko, Inst. Medical & Dental Eng.,, Kanda, Tokyo 101 (JP); JONES, Stephen, Alister, Hertfordshire EN5 4HF (GB); STRATFORD, Peter, William, London (GB)
(74) Representative: Jones, Helen Marjorie Meredith
(86) International application number: GB9401805
(87) International publication number: WO9505408

(56) References cited:
- WO-A-93/01221

## Description

The present invention relates to new polymers, processes for producing them and processes for coating surfaces with them. The invention also provides certain new monomers used to obtain the polymers and processes for their production. The polymers are useful for coating surfaces of devices and materials which come into contact with protein-containing solutions and biological fluids, and rendering the surfaces bio- and haemocompatible. Surfaces may thus be rendered suitable for prolonged contact with living tissues and body fluids and with protein-containing solutions.

Materials used in the manufacture of separation substrates and devices, blood contacting devices, contact and intraocular lenses, and other devices which are used in contact with protein-containing or biological fluids must be selected on the basis of acceptable physical and mechanical properties and compatibility with the protein-containing or biological fluid. For any given application of these materials it is usually difficult to optimise all of these considerations simultaneously and a compromise must be reached often resulting in less than optimal performance. For example, major biological problems are often encountered with materials which have otherwise optimal mechanical and physical properties. These problems often manifest themselves as undesirable deposition of biological components and in particular proteinaceous material. This protein adsorption results in blood clot formation in blood-contacting materials, the adsorption of tear components onto contact lenses resulting in deposit formation, formation of deposits on intraocular lenses and in separation media it results in blockage and failure of separation devices. Such effects lead to significant loss in operational performance and often complete rejection and failure of devices.

In the case of medical devices, for example prostheses and components of blood dialysis equipment, it is common practice to employ biocompatible polymers to form at least the surface of the devices to discourage protein adsorption. However, these materials are not perfect and reaction with the living tissues still remains a problem; for example surface-induced thrombosis is still a major difficulty, particularly where large quantities of blood are contacted with a foreign surface such as in artificial lungs and kidneys. Formation of a clot in an artificial organ has a number of adverse or even catastrophic effects including occlusion of the blood pathway in the extracorporeal system, or embolism if the clot breaks off the artificial surface and lodges in a host blood vessel. Dialysis membranes, heart valves, circulatory-assist devices, blood substitutes and artificial lungs all share this problem.

It is known that materials for use as biocompatible coatings should ideally:
(a) be capable of reproducible manufacture as pure materials;
(b) be capable of being coated onto surfaces without being degraded or adversely changed;
(c) have the requisite mechanical and permeability properties required for the specific function of the device for which they are intended;
(d) be sterilisable without adverse changes in, for example, permeability and mechanical or surface properties;
(e) not be damaged or degraded by the biological environment;
(f) not be carcinogenic.

In applications involving direct contact with blood further restrictions exist. Materials should not:
(g) induce significant platelet adhesion;
(h) interfere with the normal clotting mechanism; or
(i) cause any significant damage to the cellular elements or soluble components of the blood.

There have been many attempts to prepare biocompatible, and specifically blood compatible (i.e, haemocompatible), surfaces, which do not activate the blood coagulation process and do not promote thrombus formation. Examples of such attempts include the preparation of negatively charged surfaces, such as by use of anionic polymers or suitably oriented electret polymers, preparation of surfaces coated with the natural anticoagulant heparin or synthetic heparin analogues, preparation of surfaces with inherently low surface free energy such as by use of silicone rubber, preparation of albumin-coated surfaces, and preparation of surfaces coated with compounds such as some polymethanes which are thought to adsorb albumin preferentially from blood. All of these however have had limitations.

Our earlier patent application, WO-A-93/01221 discloses film-forming polymers which can be used to provide stable coatings on a wide variety of surfaces including polyethylene, PVC, steel and poly(imide). The polymers contain zwitterionic groups which mimic the zwitterionic structure of phospholipids such as phosphatidylcholine and sphingomyelin which are the major components of the outer membrane of all living cells and seek to provide a biocompatible surface on a coated substrate at which the deposition of proteins and cells at the substrate is minimised when the coated substrate comes into contact with a protein-containing solution or biological fluid. The polymers also contain groups which provide stable binding of the polymer to a substrate by physisorption, covalent bonding or ionic bonding.

We have also found that compounds containing phosphoryl choline groups can reduce the adhesion or growth of microorganisms such as bacteria, yeast, algae and fungi, and, in particular bacteria, at a surface. This renders the use of copolymers containing such groups advantageous in a wide variety of situations where the growth of such microorganisms is undesirable. These include industrial and domestic use as well as in the biomedical field wherever it is designed to reduce adhesion of bacteria to a surface.

We have now devised new film-forming polymers which are particularly suitable to provide stable coatings of polyurethane substrates. The polymers comprise zwitterionic groups intended to provide a biocompatible coating and groups containing a polyurethane-like chain which are capable of providing stable binding by physisorption to a polyurethane. Such polymers adhere to a polyurethane substrate surface through strong secondary valence interactions, i.e. through adsorption without the formation of covalent interactions.

It is also believed that when the polymers of the invention are blended with a conventional polyurethane they will provide a bulk material combining the physical and mechanical properties of the polyurethane and improved biocompatibility. The presence of pendant polyurethane-like groups in a copolymer of the invention may improve the compatibility of the copolymer for blending with a polyurethane.

In addition a variety of ligands may be attached to the polymers of the present invention when coated onto a substrate, or alternatively ligands may be attached to the polymers prior to coating on a substrate, e.g. when the polymer is in solution. The polymers of the present invention may therefore provide a means of attachment of such ligands: in such a case the polymers may comprise reactive groups capable of providing covalent bonding to a ligand. The term "ligand" includes, but is not limited to, specific binding agents such as immunoglobulins and fragments thereof, such as those useful for affinity separation and diagnostic applications, photosensitive and chemosensitive moieties, such as those useful for detector and sensor applications, and therapeutic agents useful for clinical applications. Other ligands include peptide fragments which may be chemically linked to a polymer of the invention, such as fragments which induce cell attachment and may therefore be used to allow the polymers of the present invention to provide cell seeding.

Coatings with increased strength and stability may be produced by incorporating in the polymers residues of comonomers which contain crosslinkable groups. After coating on a substrate surface crosslinking may then be provided by subsequent reaction of such groups.

The present invention provides a polymer of one or more radical polymerisable monomers, obtained by copolymerising a radical polymerisable ethylenically unsaturated monomer containing a zwitterionic group and a radical polymerisable ethylenically unsaturated comonomer of general formula (VI)

Y¹-Q (VI)

where Y¹ is an ethylenically unsaturated polymerisable group selected from 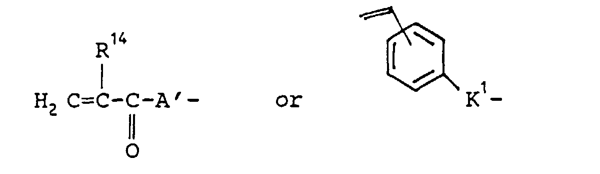 where
R¹⁴ is hydrogen or C₁-C₄ alkyl,
A' is -O- or -NR¹⁵- where R¹⁵ is hydrogen or a C₁-C₄ alkyl group or R¹⁵ is a group Q;
K¹ is a group -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in which the groups R¹⁶ are the same or different), -(CH₂)ₗO-, -(CH₂)ₗSO₃-, a valence bond and l is from 1 to 12 and R¹⁶ is hydrogen or a C₁-C₄ alkyl group; and Q is a group of formula (IXA) or (IXB)

   -R¹⁷-NRC(O)NR-R¹⁸ (IXA)

   -R¹⁷-NRC(O)O-R¹⁸ (IXB)
wherein R, (or the two groups R in (IXA), which may be the same or different each) is hydrogen or C₁₋₄ alkyl;
R¹⁷ is alkylene, arylene, alkylarylene, arylalkylene or alkylarylalkylene, cycloalkylene, alkylcycloalkylene, cycloalkyl-alkylene or alkyl-cycloalkyl-alkylene; and
R¹⁸ is alkyl, aryl or alkylaryl or cycloalkyl or alkylcycloalkyl wherein the alkyl groups may optionally be interrupted by one or more arylene or cycloalkylene groups, and wherein R¹⁸ may optionally contain one or more groups -NRC(O)NR- or -NRC(O)O- as hereinbefore defined, said group Q being capable of stably binding the polymer by physisorption to a polymeric substrate surface or capable of providing compatibility with a base polymer in a blend.

Such copolymers may be coated on a polymeric, preferably polyurethane, substrate surface so as to bind with good adhesion and are not removable in the environment in which the coated surfaces are used, e.g. in use as a coating on a blood-contacting surface. The polymeric substrate is formed of any polymer which can be shaped, usually by a melt-processing technique, and so is suitably a thermoplastic polymer. Any such polymeric substrate routinely used in blood contacting apparatus may be advantageously biocompatibilised using the new polymers. Such thermoplastic polymers include olefin polymers, eg ethylene polymers, vinyl chloride polymers, polycarbonates, polyesters, etc.

The polymers of the invention may be used to improve the biocompatibility of a surface. The extent to which such a polymer renders a substrate biocompatible may be assessed as a combination of factors such as reduction in the extent to which the surface causes blood platelet activation, protein adsorption, (for instance as judged by absorption of fibrinogen from human plasma) and reaction with C-reactive protein which is caused by the presence on the surface of isolated zwitterionic, e.g. ammonium phosphate ester groups. Preferably the polymers of the invention when coated onto a substrate, provide a reduction in platelet activation of at least 70%, more preferably at least 90%, as assessed by the assay described in WO 93/01221 compared to an untreated substrate. Alternatively, the extent to which platelets are activated by adhesion to a substrate may be assessed by Scanning Electron Microscopy. It is also preferred that the polymers of the invention, when coated onto a substrate, provide a reduction in fibrinogen absorption of at least 60% as assessed by the assay described in WO 93/01221 and a protein index of less than 1.5 x 10⁻³ compared to an untreated substrate. The protein index is defined as the ratio of the absorbency due to C-reactive protein measured in the assay described in WO 93/01221 to the reduction in fibrinogen absorption.

Polymers of the invention may also be used to reduce the tendency of microorganisms, such as bacteria, yeast, algae or fungi and in particular bacteria, to adhere to the surface. They may also be used more generally to reduce cellular adhesion at a surface. Surfaces treated with or fabricated from such polymers therefore have a wide variety of applications in the medical field such as in blood-contacting devices, and also in domestic or industrial fields wherever it is desired to reduce the adhesion of protein or microorganisms at a surface.

The nature of the pendant polyurethane-like groups in the polymers of the invention may be selected to optimise binding to a particular polymeric substrate surface which it is intended to coat with the polymer or for compatibility with a particular base polymer with which the polymer of the invention is to be blended. Thus the pendant polyurethane-like groups containing an -NRC(O)NH- or -NRC(O)O- moiety may be matched to the structure of a particular polyurethane.

The pendant groups containing an -NRC(O)NH- or -NRC(O)O- moiety, (i.e. polyurethane-like groups) may be present in the same monomer as the zwitterionic groups or they may be in separate monomer species which are copolymerised to provide the polymer of the invention.

It will be understood that throughout, where a group is referred to as capable of binding a polymer to a surface this is intended to mean stably binding, that is, the polymer is not removed from the surface to a significant extent during the normal use thereof, usually that the polymer is not removed from the surface to a significant extent in prolonged contact with blood or other aqueous liquid.

In one embodiment the present invention therefore provides a copolymer obtained by copolymerising a radical polymerisable, preferably an ethylenically unsaturated, comonomer containing a radical polymerisable comonomer of the formula VI defined above.

The polymer is a copolymer comprising residues of a radical polymerisable, preferably ethylenically unsaturated, comonomer containing a zwitterionic group and of a radical polymerisable ethylenically unsaturated comonomer containing, in addition to the radical polymerisable moiety, a group containing an -NRC(O)NR- or -NRC(O)O- moiety capable of binding to a polyurethane substrate surface by physisorption or capable of providing compatibility with a polyurethane polymer in a blend.

Optionally, the polymer may further comprise residues of one or more diluent comonomers and/or reactive comonomers to provide points of attachment for a ligand, or crosslinking.

The invention also provides a process for producing such a polymer which comprises polymerising such monomers and a process for coating a polymeric substrate surface with such a polymer, for instance a process comprising the steps of (a) polymerising such monomers to form the polymer and (b) coating the surface with the polymer so formed. Optionally, the process further comprises attaching a ligand to the polymer either in solution before coating the surface or, more preferably, when coated on the surface.

In a specific embodiment the invention further provides such polymers containing residues of a crosslinkable reactive monomer, which are uncrosslinked, when either coated on a surface or not coated on a surface and such polymers which are crosslinked when coated on a surface. The invention further provides a process of crosslinking such polymers when coated on a surface.

As yet a further feature, the present invention provides certain new monomers useful in producing the polymers of the invention, and processes for their preparation.

Monomers and comonomers which may be used in the polymers of the invention will now be described in more detail.

It is to be understood that throughout the specification (alk)acrylate, (alk)acrylic and (alk)acrylamide mean acrylate or alkacrylate, acrylic or alkacrylic and acrylamide or alkacrylamide respectively. Preferably unless otherwise stated alkacrylate, alkacrylic and alkacrylamide groups contain from 1 to 4 carbon atoms in the alkyl group thereof and are most preferably methacrylate, methacrylic or methacrylamide groups. Similarly (meth)acrylate, (meth)acrylic and (meth)acrylamide shall be understood to mean acrylate or methacrylate, acrylic or methacrylic and acrylamide or methacrylamide respectively.

### Zwitterionic Monomers

The zwitterionic monomer (or comonomer) includes within its structure both a centre of permanent positive charge and also a centre of negative charge. Typically the centre of permanent positive charge is provided by a quaternary nitrogen atom and the centre of negative change is provided by a phosphate group. Preferably the zwitterionic group is an ammonium phosphate ester group such as a phosphoryl choline group or derivative thereof.

Preferred zwitterionic comonomers are of general formula (I)

Y-B-X (I)

wherein B is a straight or branched alkylene, oxaalkylene or oligo-oxaalkylene chain optionally containing one or more fluorine atoms up to and including perfluorinated chains or, if X contains a carbon-carbon chain between B and the zwitterionic group or if Y contains a terminal carbon atom bonded to B, a valence bond;

X is a zwitterionic group and
Y is an ethylenically unsaturated polymerisable group selected from 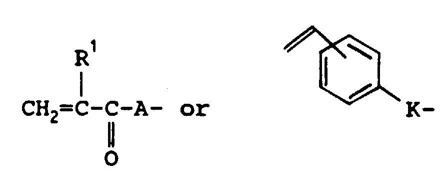 wherein:
R¹ is hydrogen or a C₁-C₄ alkyl group;
A is -O- or -NR²- where R² is hydrogen or a C₁-C₄ alkyl group or R² is -B-X where B and X are as defined above; and
K is a group -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³-, (in which the groups R³ are the same or different) -(CH₂)ₚO-, -(CH₂)ₚSO₃ -, or, optionally in combination with B, a valence bond and p is from 1 to 12 and R³ is hydrogen or a C₁-C₄ alkyl group.

The proviso on whether B may be a valence bond ensures that a centre of charge in the zwitterionic group X is not directly bonded to a heteroatom, such as an oxygen or nitrogen atom in Y.

Preferred zwitterionic are therefore of general formula (II) or (III). 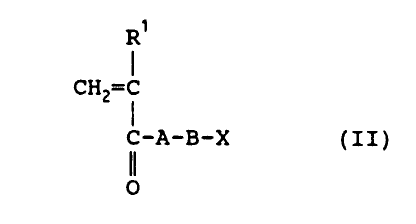 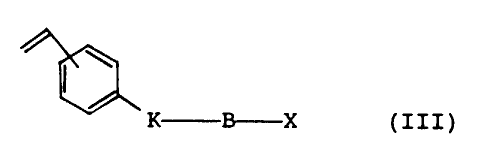 where R¹, A, B, K and X are as defined with reference to formula (I).

Preferably in the compounds of formula (II) R¹ is hydrogen, methyl, or ethyl, more preferably methyl, so that (II) is an acrylic acid, methacrylic acid or ethacrylic acid derivative.

In the compounds of formula (III) K may be a valence bond and B a group, K may be a group and B a valence bond, both K and B may be groups, or K and B may together be a valence bond. Preferably B is a group where K is a valence bond.

Where K is a group then preferably p is from 1 to 6, more preferably 1,2 or 3 and most preferably p is 1. When K is a group -(CH₂)ₚNR³-, -(CH₂)ₚNR³C(O)-, -(CH₂)ₚC(O)NR³, -(CH₂)ₚNR³C(O)O-, -(CH₂)ₚOC(O)NR³- or -(CH₂)ₚNR³C(O)NR³- then R³ is preferably hydrogen, methyl or ethyl, more preferably hydrogen.

In the compounds of formula (III) preferably the vinyl group is para to the group -K-B-X.

Preferably B is:
an alkylene group of formula -(CR⁴₂)ₐ-, wherein the groups -(CR⁴₂)- are the same or different, and in each group -(CR⁴₂)- the groups R⁴ are the same or different and each group R⁴ is hydrogen, fluorine or C₁₋₄ alkyl or fluoroalkyl, preferably hydrogen, and a is from 1 to 12, preferably 1 to 6;
an oxaalkylene group such as alkoxyalkyl having 1 to 6 carbon atoms in each alkyl moiety, more preferably -CH₂O(CH₂)₄-; or
an oligo-oxaalkylene group of formula -[(CR⁵₂)_{b}O]_{c}(CR⁵₂)_{b}- where the groups -(CR⁵₂)- are the same or different and in each group -(CR⁵₂)- the groups R⁵ are the same or different and each group R⁵ is hydrogen, fluorine or C₁₋₅ alkyl or fluoroalkyl, preferably hydrogen, and b is from 1 to 6, preferably 2 or 3 and c is from 2 to 11, preferably 2 to 5; or
if X contains a carbon-carbon chain between B and the zwitterionic group or if Y contains a terminal carbon atom, a valence bond.

Preferred groups B include alkylene, oxaalkylene and oligo-oxaalkylene groups of up to 12 carbon atoms.

In compounds of formula (III) it is preferred that K and B contain up to 12 carbon atoms in total.

Preferred zwitterionic groups X are of formula (IVA), (IVB), (IVC), (IVD) and (IVE) as defined below: monomers containing such groups may be used in combination with further monomers containing a polyurethane-like group to provide a copolymer of the invention. Of these groups, those of formula (IVB) are particularly preferred.

In addition, groups of formula (VA), (VB) and (VC) are preferred as monomers containing both a zwitterionic group and a group containing an -N(R)C(O)NR- or -N(R)C(O)0-moiety.

The groups of formula (IVA) are: 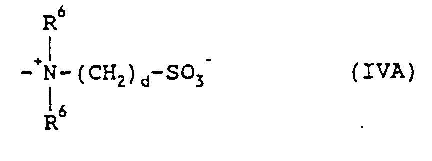 where the groups R⁶ are the same or different and each is hydrogen or C₁₋₄ alkyl or the two groups R⁶ form together with the nitrogen atom to which they are attached, a heterocyclic ring containing from 5 to 7 atoms and d is from 1 to 20.

Preferably the groups R⁶ are hydrogen or C₁₋₄ alkyl and preferably they are the same. It is also preferable that at least one of the groups R⁶ is methyl, and more preferable that the groups R⁶ are both methyl.

Preferably d is from 2 to 12, more preferably from 2 to 6, and even more preferably 2 to 4. Most preferably d is 3.

When X is a group of formula (IVA) preferably B is a group of formula -(CR⁴₂)- or -(CR⁴₂)₂-, eg. -(CH₂)- or-(CH₂CH₂)-.

Further zwitterionic groups include those corresponding to the groups of the formula (IVA) but which contain a carboxylate or phosphate group in place of the sulphonate group.

The groups of formula (IVB) are: 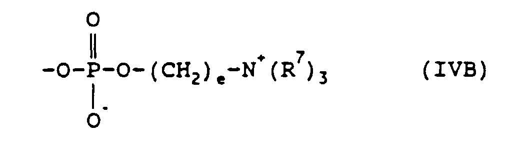 where the groups R⁷ are the same or different and each is hydrogen, C₁₋₄ alkyl, aryl, such as phenyl, or two of the groups R⁷, together with the nitrogen atom to which they are attached, form a heterocyclic ring containing from 5 to 7 atoms or the three groups R⁷, together with the nitrogen atom to which they are attached, form a fused ring structure containing from 5 to 7 atoms in each ring and e is from 1 to 20.

Preferably the groups R⁷ are hydrogen or C₁₋₄ alkyl and preferably they are the same. It is also preferable that at least one of the groups R⁷ is methyl, and more preferable that the groups R⁷ are all methyl.

Preferably e is from 2 to 12, more preferably 2 to 6 and even more preferably 2 to 4. Most preferably e is 2.

When X is a group of formula (IVB) preferably B is a group of formula -(CR⁴₂)- or -(CR⁴₂)₂-, eg. -(CH₂)- or -(CH₂CH₂)-.

The groups of formula (IVC) are: 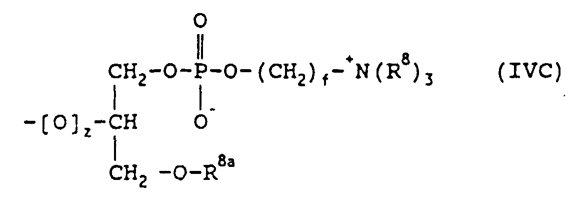 wherein the groups R⁸ are the same or different and each is hydrogen, C₁₋₄ alkyl, aryl, such as phenyl, or two of the groups R⁸, together with the nitrogen atom to which they are attached, form a heterocyclic ring containing from 5 to 7 atoms or the three groups R⁸, together with the nitrogen atom to which they are attached, form a fused ring structure containing from 5 to 7 atoms in each ring,
R^{8a} is hydrogen or, more preferably, a group -C(O)B¹R^{8b} where R^{8b} is hydrogen or methyl, preferably methyl, B¹ is a valence bond or a straight or branched alkylene, oxaalkylene or oligo-oxaalkalyene group,
f is from 1 to 20; and
if B is other than a valence bond z is 1 and if B is a valence bond z is O, if X is directly bonded to an oxygen or nitrogen atom and otherwise z is 1.

Preferably the groups R⁸ are hydrogen or C₁₋₄ alkyl and preferably they are the same. It is also preferable that at least one of the groups R⁸ is methyl, and more preferable that the groups R⁸ are all methyl.

Preferably f is from 2 to 12, more preferably 2 to 6 and even more preferably from 2 to 4. Most preferably f is 2.

Preferably B¹ is:
a valence bond;
an alkylene group of formula -(CR^{4a}₂)ₐₐ-, wherein the groups -(CR^{4a}₂)- are the same or different, and in each group -(CR^{4a}₂)- the groups R^{4a} are the same or different and each group R^{4a} is hydrogen or C₁₋₄ alkyl, preferably hydrogen, and aa is from 1 to 12, preferably 1 to 6;
an oxaalkylene group such as alkoxyalkyl having 1 to 6 carbon atoms in each alkyl moiety, more preferably -CH₂O(CH₂)₄-; or
an oligo-oxaalkylene group of formula -[(CR^{5a}₂)_{ba}O]_{ca}- where the groups -(CR^{5a}₂)- are the same or different and in each group -(CR^{5a}₂)- the groups R^{5a} are the same or different and each group R^{5a} is hydrogen or C₁₋₄ alkyl, preferably hydrogen, and ba is from 1 to 6, preferably 2 or 3, and ca is from 1 to 12, preferably 1 to 6.

Preferred groups B¹ include a valence bond and alkylene, oxaalkylene and oligo-oxaalkylene groups of up to 12 carbon atoms.

Preferably B and B¹ are the same.

When X is a group of formula (IVC) preferably B is a group of formula -[(CR⁵₂CR⁵₂)_{b}O]_{c}CR⁵₂CR⁵₂-, eg. -(CH₂CH₂O)_{c}(CH₂CH₂)-.

The groups of formula (IVD) are: 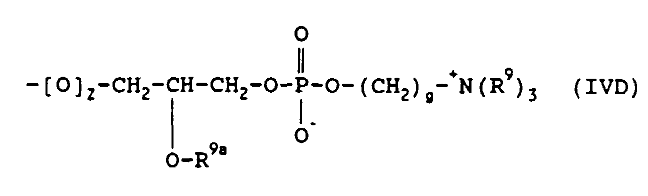 wherein the groups R⁹ are the same or different and each is hydrogen or C₁-C₄ alkyl, aryl, such as phenyl, or two of the groups R⁹, together with the nitrogen atom to which they are attached, form a heterocyclic ring containing from 5 to 7 atoms or the three groups R⁹, together with the nitrogen atom to which they are attached, form a fused ring structure containing from 5 to 7 atoms in each ring,
R^{9a} is a hydrogen or, more preferably, a group -C(O)B²R^{9b}, R^{9b} is hydrogen or methyl, preferably methyl, B² is a valence bond or a straight or branched alkylene, oxaalkylene or oligo-oxaalkylene group,
g is from 1 to 20; and
if B is other than a valence bond z is 1 and if B is a valence bond z is O if X is directly bonded to an oxygen or nitrogen atom and otherwise z is 1.

Preferably the groups R⁹ are hydrogen or C₁₋₄ alkyl and preferably they are the same. It is also preferable that at least one of the groups R⁹ is methyl, and more preferable that the groups R⁹ are all methyl.

Preferably g is from 2 to 12, more preferably 2 to 6 and even more preferably 2 to 4. Most preferably g is 2.

Preferably B² is:
a valence bond;
an alkylene group of formula -(CR^{4b}₂)_{ab}-, wherein the groups -(CR^{4b}₂)- are the same or different, and in each group -(CR^{4b}₂)- the groups R^{4b} are the same of different and each group R^{4b} is hydrogen or C₁₋₄ alkyl, preferably hydrogen, and ab is from 1 to 12, preferably 1 to 6;
an oxaalkylene group such as alkoxyalkyl having 1 to 6, carbon atoms in each alkyl moiety, more preferably -CH₂O(CH₂)₄-; or
an oligo-oxaalkylene group of formula -[(CR^{5b}₂)_{bb}O]_{cb}- where the groups -(CR^{5b}₂)- are the same or different and in each group -(CR^{5b}₂)- the groups R^{5b} are the same or different and each group R^{5b} is hydrogen or C₁₋₄ alkyl, preferably hydrogen, and bb is from 1 to 6, preferably 2 or 3, and cb is from 1 to 12, preferably 1 to 6.

Preferred groups B² include a valence bond and alkylene, oxaalkylene and oligo-oxaalkylene groups of up to 12 carbon atoms.

Preferably B and B² are the same.

When X is a group of formula (IVD) preferably B is a group of formula -[(CR⁵₂CR⁵₂)_{b}O]_{c}CR⁵₂CR⁵₂-, eg. -(CH₂CH₂O)_{c}CH₂CH₂-.

The groups of formula (IVE) are: 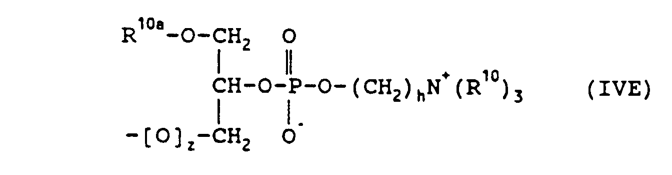 wherein
the groups R¹⁰ are the same or different and each is hydrogen, C₁₋₄ alkyl, aryl, such as phenyl, or two of the groups R¹⁰, together with the nitrogen atom to which they are attached, form a heterocyclic ring containing from 5 to 7 atoms or the three groups R¹⁰, together with the nitrogen atom to which they are attached, form a fused ring structure containing from 5 to 7 atoms in each ring,
R^{10a} is hydrogen or, more preferably, a group -C(O)B³R^{10b} where R^{10b} is hydrogen or methyl, preferably methyl, B³ is a valence bond or a straight or branched alkylene, oxaalkylene or oligo-oxaalkylene group,
h is from 1 to 20; and
if B is other than a valence bond z is 1 and if B is a valence bond z is O if X is directly bonded to the oxygen or nitrogen and otherwise z is 1.

Preferably the groups R¹⁰ are hydrogen or C₁₋₄ alkyl and preferably they are the same. It is also preferable that at least one of the groups R¹⁰ is methyl, and more preferable that the groups R¹⁰ are all methyl.

Preferably h is from 2 to 12, more preferably 2 to 6, and even more preferably 2 to 4. Most preferably h is 2.

Preferably B³ is:
a valence bond;
an alkylene group of formula -(CR^{4c}₂)_{ac}-, wherein the groups -(CR^{4c}₂)- are the same or different, and in each group -(CR^{4c}₂)- the groups R^{4c} are the same or different and each group R^{4c} is hydrogen or C₁₋₄ alkyl, preferably hydrogen, and ac is from 1 to 12, preferably 1 to 6;
an oxaalkylene group such as alkoxyalkyl having 1 to 6 carbon atoms in each alkyl moiety, more preferably -CH₂O(CH₂)₄-; or
an oligo-oxaalkylene group of formula -[(CR^{5c}₂)_{bc}O]_{cc}- where the groups -(CR^{5c}₂)- are the same or different and in each group -(CR^{5c}₂)- the groups R^{5c} are the same or different and each group R^{5c} is hydrogen or C₁₋₄ alkyl, preferably hydrogen, and bc is from 1 to 6, preferably 2 or 3, and cc is from 1 to 12, preferably 1 to 6.

Preferred groups B³ include a valence bond and alkylene, oxaalkylene and oligo-oxaalkylene groups of up to 12 carbon atoms.

Preferably B and B³ are the same.

When X is a group of formula (IVF) preferably B is a group of formula -[(CR⁵₂CR⁵₂)_{b}O)_{c}CR⁵₂CR⁵₂-, eg. -(CH₂CH₂O)_{c}CH₂CH₂-.

Further zwitterionic groups include those corresponding to the formulae (IVB) to (IVE), but which contain in place of the ammonium group -⁺NR'₃ a phosphonium group -⁺PR'ᵣ(OR')₃₋ᵣ where r is from 0 to 3, or a sulphonium group -⁺SR'₂, where R' corresponds to R⁷, R⁸, R⁹ or R¹⁰ or a group Het⁺, where Het is a nitrogen, phosphorus or sulphur-containing heterocycle, such as a pyridinium group.

Further zwitterionic groups include those which contain a sulphur atom, a group -NH- or a valence bond in place of either or both of the oxygen atoms bonding directly to the phosphorus of the phosphate group and to another atom in the groups of formulae (IVB) to (IVE).

Further zwitterionic groups are of formula (VA), (VB) and (VC). These groups also contain a group capable of binding to a polymeric substrate surface by physisorption. Monomers containing such a group are therefore particularly suitable for use in the polymers of the invention, optionally without separate comonomers containing a group capable of binding to a polymeric surface by physisorption.

As a further feature the present invention provides compounds of formula (II) or (III) containing a zwitterionic group of formula (VA), (VB) or (VC).

The groups of formula (VA) are: 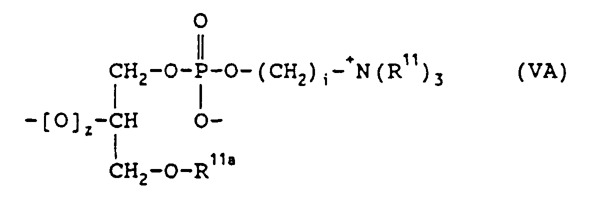 wherein
the groups R¹¹ are the same or different and each is hydrogen, C₁₋₄ alkyl, aryl, such as phenyl, or two of the groups R¹¹, together with the nitrogen atom to which they are attached, form a heterocyclic ring containing from 5 to 7 atoms or the three groups R¹¹, together with the nitrogen atom to which they are attached, form a fused ring structure containing from 5 to 7 atoms in each ring,
R^{11a} is a group of formula -C(O)R^{11b}, in which R^{11b} is a group containing an -NRC(O)NR- or -NRC(O)O- moiety capable of stably binding to a polymeric substrate surface by physisorption,
i is from 1 to 20; and
if B is other than a valence bond z is 1 and if B is a valence bond z is 0 if X is directly bonded to an oxygen or nitrogen atom and otherwise z is 1.

Preferably the groups R¹¹ are hydrogen or C₁₋₄ alkyl and preferably they are the same. It is also preferable that at least one of the groups R¹¹ is methyl, and more preferable that the groups R¹¹ are all methyl.

Preferably i is from 2 to 12, more preferably from 2 to 6, and even more preferably from 2 to 4. Most preferably i is 2.

Preferably the group R^{11b} is a group of formula (IXA) or (IXB) as defined hereinafter in relation to comonomers of formula (VII) and (VIII) which contain such a group.

The groups of formula (VB) are: 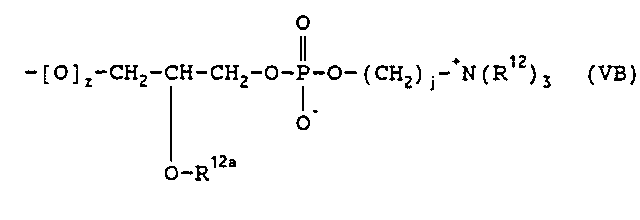 wherein
the groups R¹² are the same or different and each is hydrogen, C₁-C₄ alkyl, aryl such as phenyl, or two of the groups R¹², together with the nitrogen atom to which they are attached, form a heterocyclic ring containing from 5 to 7 atoms or the three groups R¹², together with the nitrogen atom to which they are attached, form a fused ring structure containing from 5 to 7 atoms in each ring,
R^{12a} is a group of formula -C(O)R^{12b}, in which R^{12b} is a group containing an -NRC(O)NR- or -NRC(O)O- moiety capable of stably binding to a polymeric substrate surface by physisorption;
j is from 1 to 20; and
if B is other than a valence bond, z is 1 and if B is a valence bond z is 0 if X is directly bonded to an oxygen or nitrogen atom and otherwise z is 1.

Preferably the groups R¹² are hydrogen or C₁₋₄ alkyl and preferably they are the same. It is also preferable that at least one of the groups R¹² is methyl, and more preferable that the groups R¹² are all methyl.

Preferably j is from 2 to 12, more preferably from 2 to 6, and even more preferably from 2 to 4. Most preferably j is 2.

Preferably the group R^{12b} is a group of formula (IXA) or (IXB) as defined hereinafter in relation to comonomers of formula (VII) and (VIII) which contain such a group.

The groups of formula (VC) are: 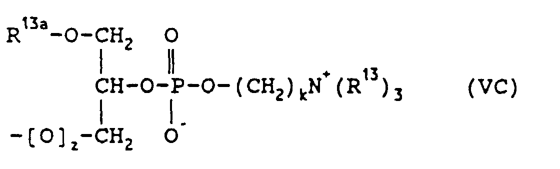 wherein
the groups R¹³ are the same or different and each is hydrogen, C₁₋₄ alkyl, aryl, such as phenyl, or two of the groups R¹³, together with the nitrogen atom to which they are attached, form a heterocyclic ring containing from 5 to 7 atoms or the three groups R¹³, together with the nitrogen atom to which they are attached, form a fused ring structure containing from 5 to 7 atoms in each ring,
R^{13a} is a group of formula -C(O)R^{13b}, in which R^{13b} is a group containing an -NRC(O)NR- or -NRC(O)O- moiety capable of stably binding to a polyurethane substrate surface by physisorption;
k is from 1 to 20; and
if B is other than a valence bond, z is 1 and if B is a valence bond z is 0 if X is directly bonded to an oxygen or nitrogen atom and otherwise z is 1.

Preferably the groups R¹³ are hydrogen or C₁₋₄ alkyl and preferably they are the same. It is also preferable that at least one of the groups R¹³ is methyl, and more preferable that the groups R¹³ are all methyl.

Preferably k is from 2 to 12, more preferably from 2 to 6, and even more preferably from 2 to 4. Most preferably k is 2.

Preferably the group R^{13b} is a group (IXA) or (IXB) as defined hereinafter in relation to comonomers of formula (VII) and (VIII) which contain such a group.

Further zwitterionic groups include those corresponding to the formulae (VA) to (VC), but which contain in place of the ammonium group -⁺NR'₃ a phosphonium group -⁺PR'ᵣ(OR')₃₋ᵣ where r is from 0 to 3, or a sulphonium group -⁺SR'₂, where R' corresponds to R¹¹, R¹², or R¹³ or a group Het⁺, where Het is a nitrogen, phosphorous or sulphur-containing heterocycle, such as a pyridinium group.

Further zwitterionic groups include those which contain a sulphur atom, a group -NH- or a valence bond in place of either or both of the oxygen atoms bonding directly to the phosphorus of the phosphate group and to another atom in formulae (VA) to (VC).

Particular examples of preferred monomers bearing a zwitterionic group are 2(methacryloyloxy)ethyl-2'(trimethylammonium)ethyl phosphate inner salt and 1[4(4'-vinylbenzyloxy)butane]-2"(trimethylammonium)ethyl phosphate inner salt.

Monomers bearing a zwitterionic group such as those of formula (II) and (III) may be prepared by conventional techniques using known reactions, for example using a suitable substituted alkyl (alk)acrylate or suitable substituted styrene as precursor. Examples of suitable substituted alkyl (alk)acrylates include dimethylaminoethyl(meth)acrylate and 2-hydroxyethyl(meth)acrylate. The preparation of such monomers is described in WO 93/01221.

Alternatively monomers of formulae (IVB) to (IVE) may be obtained by reaction of NR'₃, (R'=R⁸, R⁹, R¹⁰ or R¹¹) with a corresponding compound containing a displaceable leaving group such as halogen, alkylsulphonyloxy or arylsulphonyloxy in place of the ammonium group. Derivatives containing such displaceable groups may be obtained by the adaptation of known methods. Likewise comonomers containing other zwitterionic groups may be obtained by the adaptation of these or other methods.

Monomers of formula (II) or (III) containing a group of formula (VA), (VB) or (VC) may be prepared by direct analogy with methods for monomers containing groups of formula (IVC), (IVD) and (IVE) respectively. Similar methodology to that described in relation compounds of formula (VII) or (VIII) hereafter, may be used to introduce a group capable of binding to a polyurethane by physisorption, such as a group of formula (IXA) or (IXB).

### Comonomers containing a polyurethane-like group

The polymer comprises residues of comonomer containing a polyurethane-like group as well as the residues of the comonomer containing a zwitterionic group. Optionally, where the monomer containing a zwitterionic group also contains a polyurethane-like group, further polyurethane-like groups may be provided by additional comonomer residues containing such groups.

The comonomers containing a polyurethane-like group have general formula (VI)

Y¹-Q (VI)

where Y¹ is an ethylenically unsaturated polymerisable group selected from 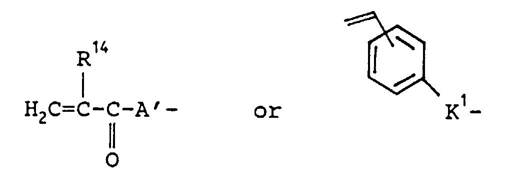 where
R¹⁴ is hydrogen or C₁-C₄ alkyl,
A' is -O- or -NR¹⁵- where R¹⁵ is hydrogen or a C₁-C₄ alkyl group or R¹⁵ is a group Q;
K¹ is a group -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in which the groups R¹⁶ are the same or different), -(CH₂)ₗO-, -(CH₂)ₗSO₃-, a valence bond and l is from 1 to 12 and R¹⁶ is hydrogen or a C₁-C₄ alkyl group; and
Q is a group of formula (IXA) or (IXB)

   -R¹⁷-NRC(O)NR-R¹⁸ (IXA)

   -R¹⁷-NRC(O)O-R¹⁸ (IXB)
wherein R, (or the two groups R in (IXA), which may be the same or different each) is hydrogen or C₁₋₄ alkyl;
R¹⁷ is alkylene, arylene, alkylarylene, arylalkylene or alkylarylalkylene, cycloalkylene, alkylcycloalkylene, cycloalkyl-alkylene or alkyl-cycloalkyl-alkylene; and
R¹⁸ is alkyl, aryl or alkylaryl or cycloalkyl or alkylcycloalkyl wherein the alkyl groups may optionally be interrupted by one or more arylene or cycloalkylene groups, and wherein R¹⁸ may optionally contain one or more groups -NRC(O)NR- or -NRC(O)O- as hereinbefore defined.

Preferred comonomers of formula (VI) bearing a group Q include those of formula (VII) and (VIII): 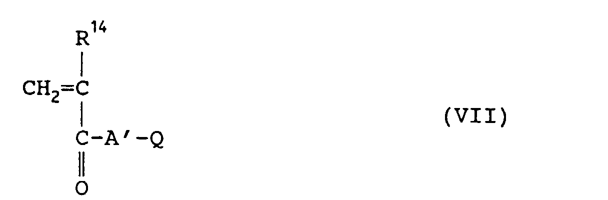 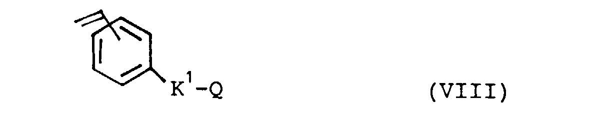 wherein:
R¹⁴, A', K¹ and Q are as defined in relation to formula (VI).

Preferably in the compounds of formula (VII) R¹⁴ is hydrogen, methyl or ethyl, more preferably methyl so that the compound of formula (VII) is preferably an acrylic acid, methacrylic acid or ethacrylic acid derivative.

In the compounds of formula (VIII) K¹ may for instance be a valence bond. Where K¹ is a group then preferably 1 is from 1 to 6, more preferably 1, 2 or 3 and most preferably l is 1. When K¹ is a group -(CH₂)ₗNR¹⁶-, -(CH₂)ₗOC(O)NR¹⁶-, -(CH₂)ₗNR¹⁶C(O)O-, -(CH₂)ₗNR¹⁶C(O)-, -(CH₂)ₗC(O)NR¹⁶- or -(CH₂)ₗNR¹⁶C(O)NR¹⁶- then R¹⁶ is preferably hydrogen, methyl or ethyl, more preferably hydrogen.

In the compounds of formula (VIII), preferably the vinyl group is para to the group -K¹-Q.

Compounds of formula (VII) and (VIII) which contain such a group (IXA) or (IXB) are provided as a further feature of the invention.

In (IXA) and (IXB), the groups R are preferably hydrogen, methyl or ethyl, more preferably hydrogen.

In R¹⁷, alkylene groups may be straight or branched, preferably straight, and preferably contain from 1 to 6 carbon atoms, for example -(CH₂)₁₋₆-. Arylene groups are preferably meta- or para-, more preferably para-, disubstituted and are preferably phenylene groups. Arylene groups may optionally be further substituted for example by one or more alkyl or alkoxy groups of 1 to 4 carbon atoms. Cycloalkylene groups may for example contain from 5 to 7 carbon atoms in the cycloalkyl ring. Preferably such groups are disubstituted cyclohexyl for example 1,4-cyclohexyl. Cycloalkylene groups may optionally be further substituted for example by one or more alkyl or alkoxy groups of 1 to 4 carbon atoms.

Particularly preferred groups R¹⁷ are -(CH₂)₁₋₆-, arylene-containing groups chosen from -(C₆H₄)-, -CH₂(C₆H₄)-, -(C₆H₄)CH₂- and -CH₂(C₆H₄)CH₂- and cycloalkylene-containing groups chosen from -(C₆H₁₀)-, -CH₂(C₆H₁₀)-, -(C₆H₁₀) CH₂- and -CH₂(C₆H₁₀)CH₂-. -CH₂CH₂- is most preferred.

In R¹⁸, alkyl groups may be straight or branched, preferably straight, and preferably contain from 1 to 10 for instance 1 to 6 carbon atoms. Aryl groups may for example by phenyl groups and arylene groups are preferably meta- or para-, more preferably para-, disubstituted and are preferably phenylene groups. Aryl and arylene groups may optionally be substituted for example by one or more alkyl or alkoxy groups of 1 to 4 carbon atoms. Cycloalkyl and cycloalkylene groups may for example contain from 5 to 7, more preferably 6, carbon atoms in the cycloalkyl ring. Preferably cycloalkylene groups are disubstituted cyclohexyl for example 1,4-cyclohexyl. Cycloalkyl and cycloalkylene groups may optionally be substituted for example by one or more alkyl or alkoxy groups of 1 to 4 carbon atoms.

Particularly preferred groups R¹⁸ are:

-(R^{18a}-NRC(O)NR)ₙ-R^{18b}

or

-(R^{18a}-NRC(O)O)ₙ-R^{18b}

where n is from 0 to 6, preferably 0 to 3, more preferably 0 or 1 and the groups R^{18a} are the same or different and each is -(CH₂)₁₋₁₀- preferably -(CH₂)₁₋₆-, an arylene-containing group which is -C₆H₄-, -(CH₂)₁₋₆(C₆H₄)-preferably -CH₂C₆H₄-, -(C₆H₄) (CH₂)₁₋₆(C₆H₄)- preferably -(C₆H₄) (CH₂) (C₆H₄)-, or -(CH₂)₁₋₆(C₆H₄) (CH₂)₁₋₆(C₆H₄)- preferably -CH₂(C₆H₄)CH₂(C₆H₄) -, or a cycloalkylene-containing group which is -C₆H₁₀-, -(CH₂)₁₋₆(C₆H₁₀)- preferably -CH₂C₆H₁₀-, -(C₆H₁₀) (CH₂)₁₋₆(C₆H₁₀)- preferably -(C₆H₁₀)(CH₂) (C₆H₁₀)-, or -(CH₂)₁₋₆(C₆H₁₀) (CH₂)₁₋₆(C₆H₁₀)- preferably -CH₂(C₆H₁₀)CH₂(C₆H₁₀)-,
and R^{18b} is -(CH₂)₁₋₉CH₃ preferably -(CH₂)₁₋₅CH₃, an aryl-containing group which is -C₆H₅, -(CH₂)₁₋₆(C₆H₅) preferably -CH₂C₆H₅, -(C₆H₄) (CH₂)₁₋₆(C₆H₅) preferably -(C₆H₄) (CH₂) (c₆H₅) or -(CH₂)₁₋₆(C₆H₄) (CH₂)₁₋₆(C₆H₅), preferably -CH₂(C₆H₄)CH₂(C₆H₅), or a cycloalkyl-containing group which -C₆H₁₁, -(CH₂)₁₋₆(C₆H₁₁) preferably -CH₂C₆H₁₁, -(C₆H₁₀)(CH₂)₁₋₆(C₆H₁₁) preferably -(C₆H₁₀)(CH₂)(C₆H₁₁) or -(CH₂)₁₋6(C₆H₁₀)(CH₂)₁₋₆(C₆H₁₁) preferably -CH₂(C₆H₁₀)CH₂(C₆H₁₁).

In the case where R¹⁷ and R¹⁸ together contain more or less than one aryl or cycloalkyl moiety, these moieties may contain the same or a different aryl or cycloalkyl ring. Preferably the aryl or cycloalkyl rings are the same.

Particular examples of compounds containing a group (IXA) or (IXB) are:
phenylaminocarbonylaminophenylmethylphenylaminocarbonylaminoethyl methacrylate;
cyclohexylaminocarbonylaminocyclohexylmethylcyclohexylaminocarbonylaminoethyl methacrylate;
butyloxycarbonylaminoethyl methacrylate;
benzyloxycarbonylaminoethyl methacrylate;
phenyloxycarbonylaminoethyl methacrylate; and
cyclohexyloxycarbonylaminoethyl methacylate.

According to a further feature the present invention provides a process for producing a compound of formula (VII) or (VIII) which contains a group (IXA) or (IXB) which process comprises reacting a corresponding compound containing, in place of the group of formula (IXA) or (IXB), a group of formula (X)

-R¹⁷-NCO (X)

where
R¹⁷ is as hereinbefore defined with
(a) a compound R¹⁸-NH₂ or an activated derivative thereof where R¹⁸ is as hereinbefore defined to produce a compound containing a group of formula (IXA); or
(b) a compound R¹⁸-OH, or an activated derivative thereof where R¹⁸ is as hereinbefore defined to produce a compound containing a group of formula (IXB).

The reactions are generally carried out in the presence of a solvent. They may be carried out at a temperature from 0 to 150°C, for example from 20 to 70°C in non-protic solvents such as DMSO, DMF, dimethylacetamide, toluene, benzene, hexane or cyclohexane. Reaction may be carried out in the presence of a catalyst such as dibutyl tin dilaurate.

Compounds R¹⁸-NH₂ and R¹⁸-OH may be produced by using known methods. Compounds containing an isocyanate group of formula (X) may likewise be produced by known methods, for example as described in Advanced Organic Chemistry, J. March, published by J. Wiley & Sons, 1985.

### Comonomers bearing a reactive group

Residues of comonomers bearing a reactive group may optionally be present in copolymers of the invention to introduce functionality for the attachment of ligand groups or to introduce crosslinkable groups. However the proportion of such comonomer residues must be sufficiently small so that the copolymer's ability to bind to a polymeric surface by physisorption or compatibility for blending with a base polymer is not substantially impaired and so that the copolymer is not rendered water soluble.

Preferred comonomers, containing a reactive group capable of providing covalent bonding to a moiety are of general formula (XI)

Y²-Q¹ (XI)

where Y² is an ethylenically unsaturated polymerisable group selected from 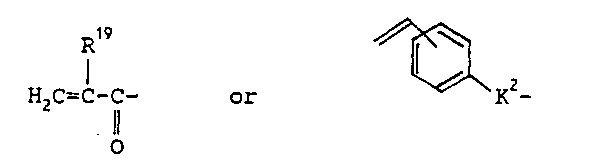 where
R¹⁹ is hydrogen or C₁-C₄ alkyl,
K² is a group -(CH₂)_{q}OC(O)-, -(CH₂)_{q}C(O)O-, -(CH₂)_{q}OC(O)O-, -(CH₂)_{q}NR²⁰-, -(CH₂)_{q}NR²⁰C(O)-, -(CH₂)_{q}C(O)NR²⁰-, -(CH₂)_{q}NR²⁰C(O)O-, -(CH₂)_{q}OC(O)NR²⁰-, -(CH₂)_{q}NR²⁰C(O)NR²⁰- (in which the groups R²⁰ are the same or different), -(CH₂)_{q}O-, or -(CH₂)_{q}SO₃-, or a valence bond and q is from 1 to 12 and R²⁰ is hydrogen or a C₁-C₄ alkyl group; and
Q¹ is a reactive group capable of reacting to provide covalent bonding.

Preferred comonomers of formula (XI) bearing a reactive group Q¹ include those of formula (XII) and (XIII) defined below.

The compounds of formula (XII) are: 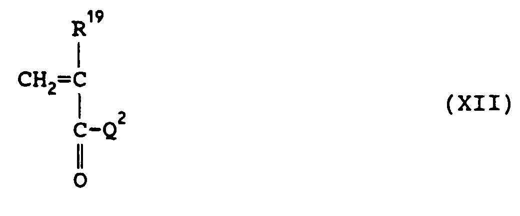 wherein:
R¹⁹ is as defined with reference to formula (XI), and Q² is a reactive group.

Preferably in the compounds of formula (XII) R¹⁹ is hydrogen, methyl or ethyl, more preferably methyl, so that the compound of formula (XII) is preferably an acrylic acid, methacrylic acid or ethacrylic acid derivative.

Preferably Q² is hydrogen, or more preferably -OH or a group of the formula:

-T-B⁷-Q³

where T is -O-, or -NR²¹- where R²¹ is hydrogen, C₁-C₄ alkyl or a group -B⁷-Q³;
B⁷ is a valence bond or, more preferably, a straight or branched alkylene, oxaalkylene or oligo-oxaalkylene chain; and
Q³ is a reactive group capable of reacting to provide covalent bonding.

Preferably B⁷ is:
an alkylene group of formula -(CR²²₂)ᵣ-, a wherein the groups -(CR²²₂)- are the same or different, and in each group -(CR²²₂)- the groups R²² are the same or different and each group R²² is hydrogen or C₁₋₄ alkyl, preferably hydrogen, and r is from 1 to 12, preferably 1 to 6;
an oxaalkylene group such as alkoxyalkyl having 1 to 6 carbon atoms in each alkyl moiety; or
an oligo-oxaalkylene group of formula -[(CR²³₂)ₛO)ₜ(CR²³₂)ₛ- where the groups -(CR²³₂)- are the same or different and in each group -(CR²³₂)- the groups R²³ are the same or different and each group R²³ is hydrogen or C₁₋₄, alkyl, preferably hydrogen, and s is from 1 to 6, preferably 2 or 3, and t is from 1 to 11, preferably 1 to 5.

Preferred reactive comonomers which are used to crosslink the comonomer, are those of formula (XII) or (XIII) in which Q², or Q⁴ contains a crosslinkable group such as a cinnamyl, epoxy, -CHOHCH₂Hal (in which Hal is a halogen atom), methylol or silyl group, an ethylenically unsaturated crosslinkable group such as an acetylenic, diacetylenic, vinylic or divinylic group, an acetoacetoxy group or chloroalkyl sulfone, preferably chloroethyl sulphone, group.

Particular examples of comonomers bearing a group capable of crosslinking include methacrolein, cinnamyl methacrylate, 2,3-epoxypropyl methacrylate, 3-chloro-2-hydroxypropyl methacrylate, hydroxymethyl methacrylamide, 3-(trimethoxysilyl)propyl methacrylate, 2-acetoacetoxyethyl methacrylate, 3-(vinylbenzyl)-2-chloroethyl sulfone.

When a polymer of the invention, containing crosslinkable groups, is coated on a substrate the polymer is in substantially uncrosslinked form. After coating, crosslinking of crosslinkable groups may be performed to increase the strength and stability of the polymer coating.

Comonomers containing a reactive group, such as compounds of formula (XII) and (XIII) may alternatively be used as comonomers which provide means for the attachment of a ligand to the copolymers of the invention.

Preferred reactive comonomers which are used to provide such means of attachment are those of formula (XII) or (XIII) where Q² or Q⁴ is a hydroxyl, amino or carboxylic acid group or an activated derivative thereof such as a succinimido, tosylate, triflate, imidazole carbonyl-amino or substituted triazine group. Preferably such a reactive group Q² or Q⁴ is separated from the copolymer chain by a spacer group, B⁷ or B⁸, of sufficient length to allow the functional group Q² or Q⁴ to interact with ligand. Alternatively such compounds may be used for the attachment of other molecules to the copolymer, such as molecules which will then interact with a ligand.

It will be appreciated that the number of sites for the attachment of such a ligand or other molecules to a surface may be varied by varying the proportion of comonomer containing reactive groups in the copolymer used to coat the surface.

Comonomers containing a reactive group, such as those of formula (XII) and (XIII) may be obtained by known methods or by the adaptation thereof.

### Diluent Comonomers

The polymers of the present invention may optionally comprise residues of a diluent comonomer, in addition to zwitterionic comonomer and comonomer containing a polyurethane-like group.

Such diluent comonomers may be used to give the polymer the desired physical and mechanical properties. They may be of any known conventional radical polymerisable, preferably ethylenically unsaturated, type compatible with other comonomer(s).

Particular examples of diluent comonomers include alkyl(alk)acrylate preferably containing 1 to 30, more preferably 1 to 12, for example 1 to 4 carbon atoms in the alkyl group of the ester moiety, such as methyl (alk)acrylate; a dialkylamino alkyl(alk)acrylate, preferably containing 1 to 4 carbon atoms in each alkyl moiety of the amine and 1 to 4 carbon atoms in the alkylene chain, e.g. 2-(dimethylamino)ethyl (alk)acrylate; an alkyl (alk)acrylamide preferably containing 1 to 4 carbon atoms in the alkyl group of the amide moiety; a hydroxyalkyl (alk)acrylate preferably containing from 1 to 4 carbon atoms in the hydroxyalkyl moiety, e.g. a 2-hydroxyethyl (alk)acrylate; or a vinyl monomer such as an N-vinyl lactam, preferably containing from 5 to 7 atoms in the lactam ring, for instance vinyl pyrrolidone; styrene or a styrene derivative which for example is substituted on the phenyl ring by one or more alkyl groups containing from 1 to 6, preferably 1 to 4, carbon atoms, and/or by one or more halogen, such as fluorine atoms, e.g. (pentafluorophenyl)styrene.

Other suitable diluent comonomers include polyhydroxyl, for example sugar, (alk)acrylates and (alk)acrylamides in which the alkyl group contains from 1 to 4 carbon atoms, e.g. sugar acrylates, methacrylates, ethacrylates, acrylamides, methacrylamides and ethacrylamides. Suitable sugars include glucose and sorbitol. Particularly suitable diluent comonomers include methacryloyl glucose or sorbitol methacrylate.

Further diluents which may be mentioned specifically include polymerisable alkenes, preferably of 2-4 carbon atoms, eg. ethylene, dienes such as butadiene, alkylene anhydrides such as maleic anhydride and cyano-substituted alkylenes, such as acrylonitrile.

Diluent comonomers may be obtained by conventional known methods.

Of the above diluent comonomers some are inert and act simply to modify the physical and mechanical properties of copolymers containing them. Others, and in particular the hydroxyalkyl(alk) acrylates and polyhydroxyl (alk) acrylates have a reactive role in addition to simply modifying physical and mechanical properties. Such comonomers contain functional groups, such as hydroxyl groups, which may react with a crosslinking group or may react with reactive groups in other molecules to attach them to the copolymer.

According to a feature of the present invention polymers of the invention may be prepared by copolymerising a comonomer containing a zwitterionic group, a comonomer containing a polyurethane-like group, a diluent and/or reactive comonomer.

Any conventional technique may be used for polymerisation, typically thermal or photochemical polymerisation. Where comonomers capable of producing crosslinking in the coated polymer film are present, the polymerisation conditions are set such that crosslinking does not occur during polymerisation. Thus, for example, actinic radiation would not be used to prepare a polymer containing a comonomer which can form crosslinks by exposure to actinic radiation.

For thermal polymerisation a temperature from 40 to 100°C, typically 50 to 80°C is used. For photochemical polymerisation actinic radiation such as gamma, U.V., visible, or microwave radiation may be used. Typically U.V. radiation of wavelength 200 to 400 nm is used.

The polymerisation is generally performed in a reaction medium, which is for instance a solution or dispersion using as a solvent for example acetonitrile, dimethyl formamide, chloroform, dichloromethane, ethyl acetate, dimethyl sulphoxide, dioxan, benzene, toluene, tetrahydrofuran, or where the polymer does not contain groups which react with protic solvents, water or an alkanol containing from 1 to 4 carbon atoms, e.g. methanol, ethanol or propan-2-ol. Alternatively, a mixture of any of the above solvents may be used.

The polymerisation may be carried out in the presence of one or more polymerisation initiators, such as benzoyl peroxide, 2,2'-azo-bis(2-methylpropionitrile) or benzoin methyl ether. Other polymerisation initiators which may be used are disclosed in "Polymer Handbook", 3rd edition, Ed. J. Brandrup and E.H. Immergut, Pub. Wiley-Interscience, New York, 1989.

Generally the copolymerisation is performed for 1 to 72 hours, preferably 4 to 48, for instance 16 to 24 hours, and under an inert atmosphere of for example nitrogen or argon. The polymer is generally purified by dialysis, precipitation in a non-solvent (e.g. diethyl ether or acetone) or ultrafiltration. The resulting polymer is generally dried under vacuum, eg. for 5 to 72 hours and has a molecular weight from 10,000 to 10 million, preferably from 20,000 to 1 million.

The precise proportion and nature of the various comonomers used to prepare a copolymer according to the present invention comprising residues of a zwitterionic comonomer and a comonomer containing a polyurethane-like group may be adjusted to provide a copolymer which is particularly suitable for coating a particular surface or compatibility with a particular base polymer in a blend. Thus the proportion and nature of comonomer containing a polyurethane-like group may be adapted to provide efficient physisorption at a particular polymeric surface or compatibility with a particular base polymer in a blend. Similarly the proportion of the comonomer containing a zwitterionic group and of diluent and/or reactive comonomer may be adapted to provide the desired biocompatibility and physical and mechanical properties. It will be appreciated that to obtain the desired combination of properties more than one type of comonomer containing a zwitterionic group, comonomer containing a group capable of stably binding the polymer to a surface or crosslinkable and/or diluent comonomer may be used.

Similarly, in polymers comprising residues of a monomer containing a zwitterionic group and a polyurethane-like group, the nature of these groups may be adjusted to provide the desired biocompatibility and efficient binding at a particular surface, as well as desired physical and mechanical properties. Where, in addition, a diluent and/or crosslinkable comonomer is used the nature of the diluent and/or crosslinkable comonomer and the proportions of the comonomers may be likewise adjusted. It will again be appreciated that to obtain the desired combination of properties more than one type of monomer containing a zwitterionic group and a polyurethane-like group and/or more than one type of crosslinkable and/or diluent comonomer may be used.

Where different comonomers are used to provide the zwitterionic group and the physisorption, then preferably the molar ratio in the copolymer of zwitterionic comonomer residues to comonomer residues containing a polyurethane-like group is from 5:95 to 90:10, more preferably 10:90 to 75:25. Preferably the molar ratio of zwitterionic comonomer residues to comonomer residues containing a polyurethane-like group and diluent comonomer residues is from 5:95 to 80:20, more preferably 10:90 to 50:50.

The copolymer preferably comprises from 5% to 75%, more preferably 10% to 60%, by mole residues of diluent monomer and/or from 0.1% to 20%, more preferably 1% to 10%, by mole residues of reactive comonomer, provided that where residues of both diluent and reactive comonomer are present, they do not exceed in combination 75%, preferably 60% by mole.

In addition the monomer or comonomer composition may comprise further components such as a polymerisation initiator, chain transfer agent, acid, base, surfactant, emulsifier or catalyst of conventional type each in an amount from 0.1% to 5%, typically from 0.2% to 3% and preferably about 0.5%, by weight each relative to the total weight of the monomers.

As a further feature the present invention provides a process for biocompatibilising a polymeric substrate surface which comprises coating the surface with a polymer according to the present invention. Various types of surfaces may be coated depending upon the nature of the groups in the polymer capable of binding it to the surface.

Treatment with such a polymer is generally carried out by coating the surface with a solution, dispersion (including a microdispersion) of the polymer, generally in an alcoholic, aqueous, organic or halogenated solvent or a mixture thereof, e.g. methanol, ethanol, dichloromethane or freon. The treatment is generally carried out at ambient or elevated temperature, such as from 5 to 60°C.

Polymeric substrates may be coated with polymers of the invention by known techniques, such as dip-coating, spray-coating, web-coating or spin coating.

The polymers of the present invention are particularly suitable for treating the following polyurethane substrates:
Esthane®, Ostamer®, Biomer®, Pellethane®, Lycra®, Spandex®, Mitrathane®, Cardiothane® and Tecoflex®.

After coating the polymer may be crosslinked if it contains the residues of crosslinkable comonomer by known method for crosslinking the specific crosslinkable groups which are present. Crosslinking may, for instance, be introduced thermally, using actinic radiation, using reactive gases for example ammonia by changing the pH, using difunctional additives or by using activation chemistries for example by known methods as described in "Methods in Enzymology, volume 135, Immobilised Enzymes and Cells, part B", Ed. K. Mosbach, Academic Press Inc, New York, 1987. This activation may be performed on the dry coating, in the cases of thermal radiation or gas treatment. Alternatively for cases where the pH needs to be changed or additives need to be included, activation may be performed on the coated material in a solution which does not remove the coating.

Polymeric materials having surfaces coated according to the present invention can be used as components for implants or prostheses for the human or animal body, particularly where these implants or prostheses are to come into direct physical contact with blood and where biocompatibility and particularly haemocompatibility are required e.g. in heart valves. They can also be used in the construction of membranes and other devices that are to be brought into contact with blood or other body fluids on an extra-corporeal basis, for example in heart-lung machines or artificial kidneys.

Additionally the polymers of the invention can be used to coat materials employed in down stream processing applications e.g. separation membranes and process equipment and tubing. In particular the materials of the invention can be used to modify the surface properties of biofiltration membranes in bioreactors and fermentation systems, where the membranes come into direct contact with complex biological solutions containing e.g. proteins, polysaccharides, fats and even whole cells. They may also be used in membranes which come into contact with microorganisms, such as bacteria, to prevent adhesion of such microorganisms. The polymers of the invention are particularly useful in reducing membrane fouling by the components of a process solution.

When the polymers of the present invention are used to coat the surface of a material which is then used in the construction coat of finished devices, it may be necessary to take precautionary steps to ensure that the coated surface is not damaged and the effectiveness of the treatment reduced before the finished device is produced.

In addition, the polymers of the present invention can be used to coat finished implants, prostheses, membranes, catheters, and other devices which are coated with a polymer according to the present invention to impart biocompatibility to the article.

The invention thus also provides a finished device comprising a polymeric substrate surface having a coating thereon of a polymer of the present invention.

According to a further feature of the present invention, the polymers of the invention may be blended with a base polymer having desirable physical and/or mechanical properties to provide a blend combining the physical and/or mechanical properties of the base polymer with improved biocompatibility.

The present invention therefore further provides a blend comprising (A) a polymer according to the present invention and (B) a base polymer and a process for producing such a blend which comprises blending the polymers (A) and (B).

In particular such blends may comprise as base polymer a polyurethane (B), such as Ethane®, Ostamer®, Biomer®, Pellethane®, Lycra®, Spandex®, Mitrathane®, Cardiothane® or Tecoflex®.

Generally such blends will contain from 1 to 90% by weight of polymer (A) containing pendant zwitterionic groups and from 99 to 10% of base polymer (B) having desirable physical and/or mechanical properties. The precise proportions of the polymers (A) and (B) will depend upon the compatibility of the two polymers for blending and, it may be necessary to test the polymers together for their compatibility. This may be achieved by blending different proportions of the polymers (A) and (B) to obtain a blend with the desired balance of mechanical and physical properties as well as biocompatibility. In particular, the proportions of the two polymers may be adjusted so as to obtain desired impact resistance, tensile strength, flexural modulus, low temperature brittleness, friction coefficient film permeability, film tear resistance, film shrinkage, surface and volume resistivity, surface wettability and/or contact angle.

The minimum quantity of polymer of the invention (A) will depend upon the particular polymer (B), the content of zwitterionic groups in the polymer (A) and the desired use of the blend. However, the content of polymer (A) should be sufficient to provide a detectable modification to the biocompatible properties of the blend compared to the unblended polymer (B). Preferably the blend will contain at least 1%, more preferably 10% and still more preferably 30% of polymer (A).

In addition, the blends of the present invention may further comprise conventional additives used in polymeric materials such as plasticisers, fillers, colourants, UV absorbers, anti-oxidants and/or preservatives, such as biocides, which may be included in conventional amounts so as to be compatible with the polymers present in the blend.

The blending of the two polymers (A) and (B) may be carried out by conventional techniques for blending polymeric materials, including known solid state physical mixing techniques such as roll-milling, Banbury mixing, screw extrusion and disk compounding. Alternatively, blending may be carried out using dispersions and/or solutions of the polymers (A) and (B) in an organic solvent and the solvent removed by evaporation. Such blending may be carried out using conventional liquid phase blending techniques such as high or low shear mixers.

The invention further provides a shaped article formed from a blend of polymer of the invention and a base polymer. Such articles may be formed in a conventional manner, for example by extrusion or injection moulding or other moulding techniques and/or by machining as necessary for the desired end shape appropriate to the nature of the particular material in question.

In particular, such blends may be suitable for use in surgical implants, bioseparation apparatus, blood carrier bags, dialysis membranes, blood oxygenator films, tubing, connectors, stoppers, closures for diagnostic catheters, surgical drapes and tapes, and encapsulants for bio-medical applications. They may in particular be used in the production of prostheses, artificial hearts and vascular surfaces.

The following figures illustrate the present invention:

Figure 1 shows the structure of two polyurethanes, Pellethane and TM3 which have been used as substrates onto which copolymers of the present invention have been coated as described in example 7.

Figures 2 and 3 show scanning electronmicrographs of the products of Example 8.

The present invention will now be further illustrated by the following Examples:-

### EXAMPLE 1

### Butyloxycarbonylaminoethyl-methacrylate

Isocyanate ethyl methacrylate (0.1 mol, 15.5 g), n-butanol (0.105 mol, 7.78 g), toluene (150 mL) and a small amount of dibutyltin dilaurate were stirred at 60°C for 4.5 hours. After the reaction, toluene was removed with an evaporator and butyloxycarbonylaminoethyl-methacrylate was obtained by distillation under reduced pressure.

¹H-NMR:(CDCl₃, σ in ppm) 0.83-0.88 (α-CH₃, 3H), 1.26-1.55(CH₂ and CH₃ in side chain, 7H), 3.40-3.43(CH₂-N, 2H), 3.95-4.05(NCOO-CH₂, 2H), 4.10-4.17(CCOO-CH₂, 2H), 4.80-4.95(-NHCOO-, 1H), 5.52-5.53(C=CH, 1H), 6.05-6.06(C=CH, 1H); IR:(cm⁻¹) 3200-3400(-NHCOO-), 2800-3000(CH₂ and CH₃), 1720(C=0), 1640(C=CH2), 1580(-NHCOO-), 1100-1260(-COO-). Elemental analysis: found(calc.). C: 57.33 (57.62), H: 8.37 (8.35), N:5.83 (6.11). B. pt 132-135°C at 2 mm Hg.

### EXAMPLE 2

### Benzyloxycarbonylaminoethyl-methacrylate

Benzyloxycarbonylaminoethyl-methacrylate was prepared by a method analogous to that of Example 1 but using benzyl alcohol (0.105 mol, 11.3 g) in place of n-butanol and a reaction time of 3 hours. The reaction mixture was cooled to 0°C and the product obtained by crystallisation.

¹H-NMR:(CDCl₃, σ in ppm) 1.85-1.90 (α-CH₃, 3H), 3.44-3.45(CH₂-N, 2H), 3.95-4.05(NCOO-CH₂, 2H), 4.15-4.17(CCOO-CH₂, 2H), 4.95-4.98(-NHCOO-, 1H), 5.00-5.04(NCOCH₂-, 2H), 5.50-5.51(C=CH, 1H), 6.00-6.03(C=CH, 1H); 7.17-7.29 (C₆H₅-, 5H) ; IR:(cm⁻¹) 3250-3350(-NHCOO-), 2800-3000(CH₂ and CH₃), 17.20(C=O), 164 (C=CH2), 1600(aromatic ring), 1580 (-NHCOO-), 1100-1260(-COO-). Elemental analysis: found(calc.). C: 63.99 (64.14), H: 6.50 (6.51), N: 5.30 (5.32), M. pt 36.3°C.

### EXAMPLE 3

### Phenyloxycarbonylaminoethyl-methacrylate

Phenyloxycarbonylaminoethyl-methacrylate was prepared by a method analogous to that of Example 2 but using phenol (0.105 mol, 9.88 g) in place of benzyl alcohol and a reaction time of 19.5 hours. The reaction mixture was cooled to 0°C and the product obtained by crystallisation.

¹H-NMR:(CDCl₃, σ in ppm) 1.97 (α-CH₃, 3H), 3.57-3.60(CH₂-N, 2H), 4.29-4.31(CCOO-CH_{2,} 2H), 5.35 (-NHCOO-, 1H), 5.61-5.62(C=CH, 1H), 6.16(C=CH, 1H), 7.11-7.37 (C₆H₅-, 5H) ; IR:(cm⁻¹) 3280-3380(-NHCOO-), 2800-3000(-CH₂- and -CH₃), 1720(C=0), 1640(C=CH2) 1580(-NHCOO-), 1500(aromatic ring), 1100-1260(-COO-). Elemental analysis: found (calc.). C: 62.60 (62.64) , H: 5.09 (6.06), N: 5.49 (5.62), M. pt 111.0°C.

### EXAMPLE 4

### Poly(2(methacryloyloxy)ethyl)-2-(trimethyl ammonium)ethyl phosphate inner salt - co-butyl methacrylate-co-butyloxycarbonylaminoethyl-methacrylate (36:53:11)

2(Methacryloyloxy ethyl)-2'(trimethylammonium) ethyl phosphate inner salt, butylmethacrylate, butyloxycarbonylaminoethyl-methacrylate in a molar ratio 30:60:10 were dissolved in ethanol (30 ml) at a concentration of 1 M in a glass ampoule. 2,2'-azobisisobutyronitrile (AIBN) was dissolved in the solution at 1 mM. Argon was bubbled through the solution to remove oxygen and the glass ampoule was sealed. Reaction was carried out at 60°C for 4 hours. The reaction mixture was allowed to cool and poured into a mixture of diethyl ether: N,N-dimethyl formamide (9:1 vol) to remove unreacted monomer and precipitate polymer. The precipitate was filtered and dried under reduced pressure. Yield 40.8% of polymer, Molecular weight 38,000, determined by GPC with poly(oxyethylene) standard.

The ratio of the comonomer residues in the copolymer was determined by elemental analysis from phosphorus content (in the phosphorus inner salt residues) and nitrogen content (in phosphorus inner salt and urethane residues).
IR:(cm⁻¹) 3000-3500(-NHCOO-), 2800-2900(CH₂, CH₃), 1740(C=0), 1200-1300(-COO-), 1000-1200(-OPO(=O)(O')-)

### EXAMPLE 5

### Poly(2(methacryloyloxy)ethyl)-2-(trimethyl ammonium) ethyl phosphate inner salt -co-butyl methacrylate-co-benzyloxycarbonylaminoethyl-methacrylate (26:63:9)

By a procedure analogous to that of Example 4 but using benzyloxycarbonylaminoethyl-methacrylate, in place of butyloxycarbonylaminoethyl-methacrylate polymer was obtained in 55.6% yield. The polymer showed a molecular weight of 59,000 as determined by GPC with poly(oxyethylene) standard and the content of the comonomer residues was determined as in Example 4. Absorption peaks similar to those in the IR spectrum given in Example 4 were observed.

### EXAMPLE 6

### Poly(2(methacryloyloxy)ethyl)-2-(trimethyl ammonium) ethyl phosphate inner salt -co-butyl methacrylate-co-phenyloxycarbonylaminoethyl-methacrylate (30:58:12)

By a procedure analogous to that of Example 4 but using phenyloxycarbonylaminoethyl-methacrylate in place of butyloxycarbonylaminoethyl-methacrylate polymer was obtained in 43.9% yield. The polymer showed a molecular weight of 41,000 as determined by GPC with poly(oxyethylene) standard and the content of the comonomer residues was determined as in Example 4. Absorption peaks similar to those in the IR spectrum given in Example 4 were observed.

### EXAMPLE 7

### Coating of polyurethane membrane

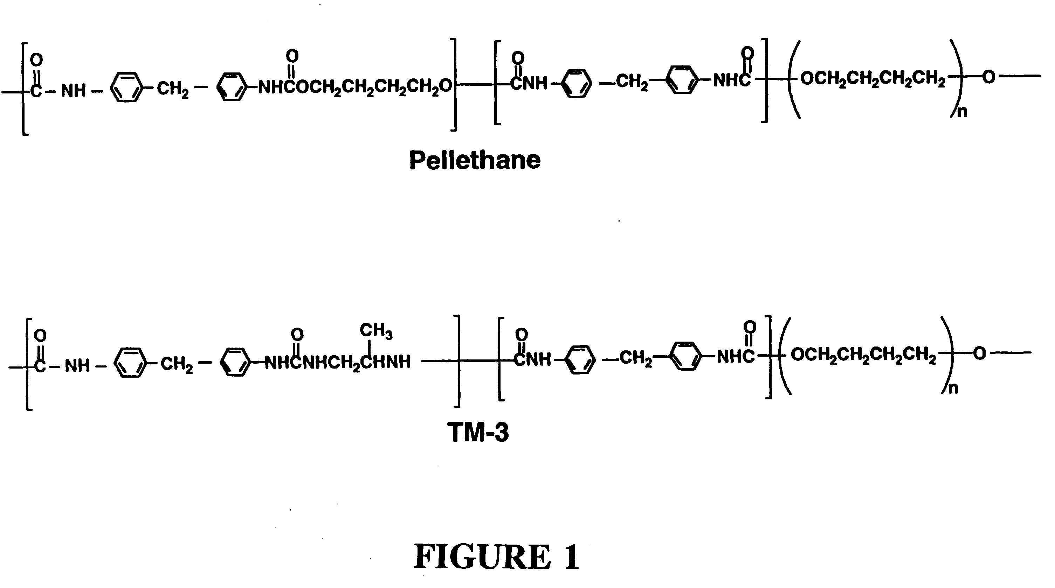

Polyurethane membranes were prepared by solvent evaporation. The polyurethane was dissolved in tetrahydrofuran and the solution poured onto a teflon plate. The solvent was allowed to evaporate at room temperature and the membrane formed was dried under reduced pressure at 40°C removing residual solvent. The membrane thus produced had a thickness of 0.3 mm. Membranes were prepared using the polyurethanes Pellethane and TM-3 whose structures are shown in Figure 1.

Polyurethane Pellethane and TM-3 membranes were coated with the copolymers of Examples 4 to 6 and, as a comparison, a copolymer of 2(methacryloyloxyethyl)-2'(trimethylammonium) ethyl phosphate inner salt: butyl methacrylate (3:7) produced by an analogous procedure. The coating copolymer was dissolved in ethanol and a polyurethane membrane immersed in the solution for 1 minute and then dried at room temperature for 16 hours. The procedure was repeated twice and the membrane was then dried under reduced pressure at room temperature.

The surfaces of the coated membranes were analysed by x-ray photoelectron spectroscapy (XPS, Shimadzu ESCA-750, Kyoto). XPS analysis was also conducted on coated membranes which had been immersed in water, ethanol or 40% aqueous ethanol for 3 hours.

XPS analysis showed the presence of a phosphorus peak and nitrogen peaks attributable to the phosphate ester inner salt groups in the coating copolymer on the membrane's surface. These were shown to be retained upon immersion in water, ethanol or aqueous ethanol for the copolymers of Examples 4 to 6 to a significantly greater extent than on a membrane coated with the comparison copolymer with butylmethacrylate. These results are confirmed by the ratio of phosphorus to carbon obtained by XPS analysis for Pellethane membranes coated with copolymers of Examples 4 to 6 and the comparison copolymer (C), in the following table:

| Example | No Immersion | H₂O Immersion | P/C Ratio EtOH Immersion | EtOH/H₂O Immersion |
|---|---|---|---|---|
| 4 | 0.036 | 0.035 | 0.026 | 0.029 |
| 5 | 0.023 | 0.022 | 0.019 | 0.022 |
| 6 | 0.037 | 0.028 | 0.028 | 0.030 |
| C | 0.034 | 0.023 | 0.016 | 0.018 |

These results indicate that copolymers of the invention which contain pendant polyurethane-like groups adhere to polyurethane substrates significantly more strongly than the comparison copolymers which do contain pendant zwitterionic and butyl groups but no pendant polyurethane-like groups.

### EXAMPLE 8

### Blood Contact Test

Disks (15 mm in diameter) of polyurethane membrane, produced by Example 7, were placed in a cell culture plate and fixed in place with a silicone ring. The membrane surface was equilibrated using 1 ml of phosphate buffered saline (PBS) (pH 7.4, ionic strength 0.15 M) which was added and incubated for 15 hours. The PBS was removed and 1 ml of rabbit citrated whole blood or platelet-rich plasma (PRP) was contacted with the membrane and maintained at 37°C for 60 minutes. The blood or RPR was then removed with a vacuum aspirator and the membrane rinsed three times with 1 ml of PBS. 1 ml of 2.5 vol% glutaraldehyde in PBS was added and maintained at room temperature for 2 hours to fix the blood component to the membrane. After rinsing with distilled water, the membranes were freeze-dried and the surfaces observed with scanning electron microscopy following gold spattering on the membrane surface.

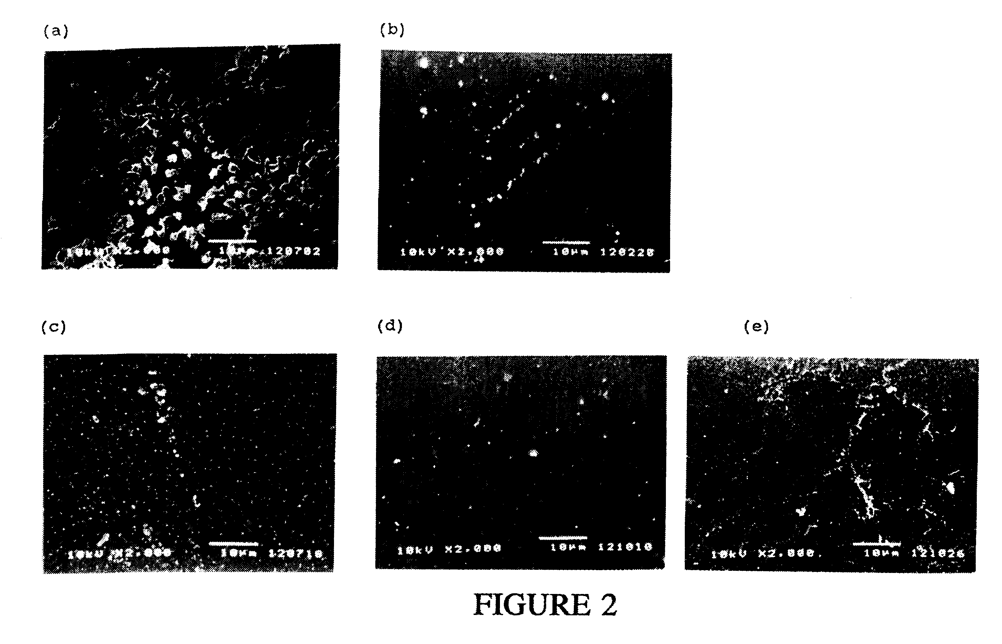

Figure 2 shows scanning electronmicrographs of Pellethane membranes after contact with rabbit platelet-rich plasma for 60 minutes. (a) shows a membrane untreated with coating copolymer, (b) shows a membrane treated with a copolymer of (2(methacryloyloxy ethyl)-2'(trimethylammonium)ethyl phosphate inner salt): - butylmethacrylate (3:7) as a comparison, and (c), (d) and (e) show membranes coated with copolymers of the invention prepared in accordance with Examples 4, 5 and 6 respectively.

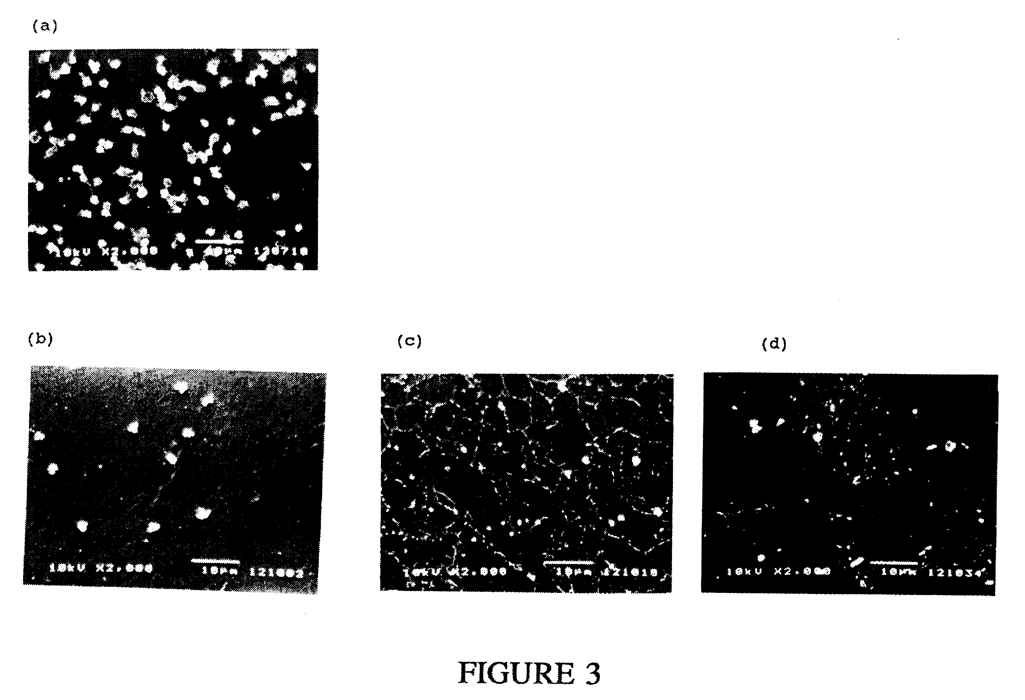

Figure 3 shows scanning electronmicrographs of polyurethane TM-3 membranes after contact with rabbit platelet-rich plasma for 60 minutes. (a) shows a membrane untreated with coating copolymer and (b), (c) and (d) show membranes coated with copolymers of the invention prepared in accordance with Examples 4, 5 and 6. The micrographs show a substantially greater deposit of platelets on the uncoated membranes (a) than those which have been coated with copolymers obtained by Examples 4 to 6, [Fig 2 (c)-(e) and Fig 3 (b)-(d)].

## Claims

1. A polymer of one or more radical polymerisable monomers, obtained by copolymerising a radical polymerisable ethylenically unsaturated monomer containing a zwitterionic group and a radical polymerisable ethylenically unsaturated comonomer of general formula (VI)
Y¹-Q (VI)
where Y¹ is an ethylenically unsaturated polymerisable group selected from 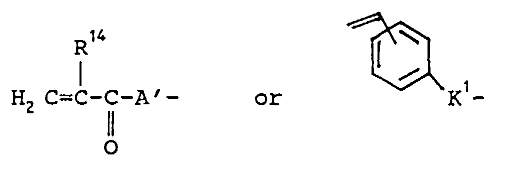 where R¹⁴ is hydrogen or C₁-C₄ alkyl,
A' is -O- or -NR¹⁵- where R¹⁵ is hydrogen or a C₁-C₄ alkyl group or R¹⁵ is a group Q;
K¹ is a group -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in which the groups R¹⁶ are the same or different), -(CH₂)ₗO-, -(CH₂)ₗSO₃-, a valence bond and l is from 1 to 12 and R¹⁶ is hydrogen or a C₁-C₄ alkyl group; and Q is a group of formula (IXA) or (IXB)
-R¹⁷-NRC(O)NR-R¹⁸ (IXA)
-R¹⁷-NRC(O)O-R¹⁸ (IXB)
wherein R, (or the two groups R in (IXA), which may be the same or different each) is hydrogen or C₁₋₄ alkyl;
R¹⁷ is alkylene, arylene, alkylarylene, arylalkylene or alkylarylalkylene, cycloalkylene, alkylcycloalkylene, cycloalkyl-alkylene or alkyl-cycloalkyl-alkylene; and
R¹⁸ is alkyl, aryl or alkylaryl or cycloalkyl or alkylcycloalkyl wherein the alkyl groups may optionally be interrupted by one or more arylene or cycloalkylene groups, and wherein R¹⁸ may optionally contain one or more groups -NRC(O)NR- or -NRC(O)O- as hereinbefore defined, said group Q being capable of stably binding the polymer by physisorption to a polymeric substrate surface or capable of providing compatibility with a base polymer in a blend.

2. A polymer according to claim 1 comprising residues of one or more diluent comonomers and/or reactive comonomers to provide points of attachment for a ligand, or crosslinking.

3. A polymer according to claim 1 or claim 2 in which the centre of permanent positive charge in the zwitterionic monomer is provided by a quaternary nitrogen atom and the centre of negative charge is provided by a phosphate group.

4. A polymer according to any preceding claim in which the zwitterionic monomer has the general formula (I)
Y-B-X (I)
wherein
B is a straight or branched alkylene, oxaalkylene or oligo-oxaalkylene chain optionally containing one or more fluorine atoms up to and including perfluorinated chains or, if X contains a carbon-carbon chain between B and the zwitterionic group or if Y contains a terminal carbon atom bonded to B, a valence bond;
X is a zwitterionic group and
Y is an ethylenically unsaturated polymerisable group selected from 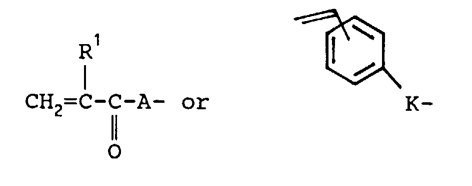 wherein:
R⁴ is hydrogen or a C₁-C₄ alkyl group;
A is -O- or -NR²- where R² is hydrogen or a C₁-C₄ alkyl group or R² is -B-X where B and X are as defined above; and
K is a group -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³-, (in which the groups R³ are the same or different) -(CH₂)ₚO-, -(CH₂)ₚSO₃ -, or, optionally in combination with B, a valence bond and p is from 1 to 12 and R³ is hydrogen or a C₁-C₄ alkyl group.

5. A polymer according to claim 4 in which the monomer has the formula 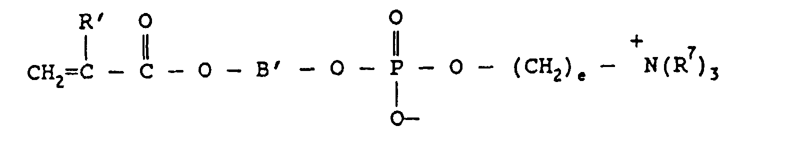 where R' is hydrogen or methyl, B' is alkylene having up to 12 carbon atoms, the groups R⁷ are the same or different and each is hydrogen, C₁₋₄ alkyl, aryl, such as phenyl, or two of the groups R⁷, together with the nitrogen atom to which they are attached, form a heterocyclic ring containing from 5 to 7 atoms or the three groups R⁷, together with the nitrogen atom to which they are attached, form a fused ring structure containing from 5 to 7 atoms in each ring and e is from 1 to 20.

6. A polymer according to claim 5 in which R⁷ are each hydrogen or C₁₋₄alkyl, preferably are all methyl.

7. A polymer according to any preceding claim in which the said co-monomer has the formula

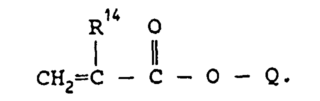

8. A polymer according to claim 7 in which R is hydrogen, methyl or ethyl, preferably hydrogen.

9. A polymer according to any preceding claim comprising residues of a comonomer bearing a reactive group of the general formula (XI)
Y²-Q¹ (XI)
where Y² is an ethylenically unsaturated polymerisable group selected from 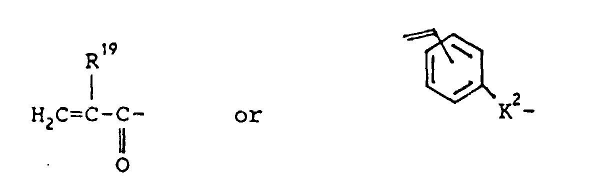 where
R¹⁹ is hydrogen or C₁-C₄ alkyl,
K² is a group -(CH₂)_{q}OC(O)-, -(CH₂)_{q}C(O)O-, -(CH₂)_{q}OC(O)O-, -(CH₂)_{q}NR²⁰-, -(CH₂)_{q}NR²⁰C(O)-, -(CH₂)_{q}C(O)NR²⁰-, -(CH₂)_{q}NR²⁰C(O)O-, -(CH₂)_{q}OC(O)NR²⁰-, -(CH₂)_{q}NR²⁰C(O)NR²⁰- (in which the groups R²⁰ are the same or different), -(CH₂)_{q}O-, or -(CH₂)_{q}SO₃-, or a valence bond and q is from 1 to 12 and R²⁰ is hydrogen or a C₁-C₄ alkyl group; and
Q¹ is a reactive group capable of reacting to provide covalent bonding, or crosslinking, selected from groups containing cinnamyl, epoxy, -CHOHCH₂Hal (in which Hal is a halogen atom), methylol or silyl group, an ethylenically unsaturated crosslinkable group such as an acetylenic, diacetylenic, vinylic or divinylic group, an acetoacetoxy group or chloroalkyl sulfone, preferably chloroethyl sulphone, group or a hydroxyl, amino or carboxylic acid group or an activated derivative thereof such as a succinimido, tosylate, triflate, imidazole carbonyl-amino or substituted triazine group.

10. A process for preparing a polymer according to claim 1 by copolymerising the said monomer containing a zwitterionic group and the said comonomer containing the group Q which includes a polyurethane-like group, optionally with a diluent and/or reactive comonomer.

11. A process according to claim 10 comprising 5 to 75% by mole, residues of a diluent monomer and/or 0.1 to 20%, by mole, of a reactive comonomer.

12. A product comprising a polymeric substrate coated with or a base polymer blended with a polymer according to any of claims 1 to 9.

13. A product according to claim 12 in which the polymeric substrate or base polymer is a polyurethane.

14. A product according to claim 12 or 13 which is a device to be contacted with blood or protein or microorganisms.

15. A process for biocompatibilising a polymeric substrate by coating its surface with a polymer according to any of claims 1 to 9.

16. A process according to claim 15 in which the polymeric substrate comprises a polyurethane.

17. A process according to claim 15 or 16 in which the platelet activation of the surface of the substrate is reduced by at least 70% by the coating.

18. A process according to any of claims 15 to 17 in which the fibrinogen absorption of the surface of the substrate is reduced by at least 60% by the coating.

19. A process in which a base polymer and a polymer according to any of claims 1 to 9 are blended together.

20. A process according to claim 19 in which the base polymer is a polyurethane.

21. A process according to claim 19 or 20 in which the blend is shaped to form an article.

22. A compound of the formula II or III. 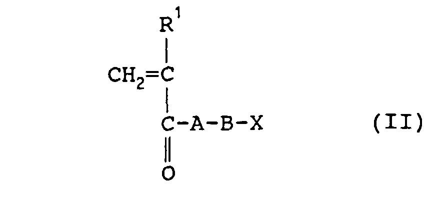 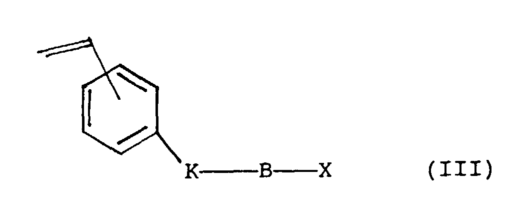 wherein
B is a straight or branched alkylene, oxaalkylene or oligo-oxaalkylene chain optionally containing one or more fluorine atoms up to and including perfluorinated chains or, if X contains a carbon-carbon chain between B and the zwitterionic group or if Y contains a terminal carbon atom bonded to B, a valence bond;
R¹ is hydrogen or a C₁-C₄ alkyl group;
A is -O- or -NR²- where R² is hydrogen or a C₁-C₄ alkyl group or R² is -B-X where B is as defined above; and
K is a group -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³-, (in which the groups R³ are the same or different) -(CH₂)ₚO-, -(CH₂)ₚSO₃ -, or, optionally in combination with B, a valence bond and p is from 1 to 12 and R³ is hydrogen or a C₁-C₄ alkyl group, and
X is selected from groups VA, VB and VC; 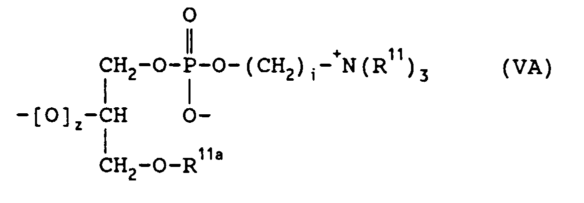
wherein the groups R¹¹ are the same or different and each is hydrogen, C₁₋₄ alkyl, aryl, such as phenyl, or two of the groups R¹¹, together with the nitrogen atom to which they are attached, form a heterocyclic ring containing from 5 to 7 atoms or the three groups R¹¹, together with the nitrogen atom to which they are attached, form a fused ring structure containing from 5 to 7 atoms in each ring,
R^{11a} is a group of formula -C(O)R^{11b}, in which R^{11b} is a group containing an -NRC(O)NR- or -NRC(O)O- moiety capable of stably binding to a polymeric substrate surface by physisorption,
i is from 1 to 20; and
if B is other than a valence bond z is 1 and if B is a valence bond z is 0 if X is directly bonded to an oxygen or nitrogen atom and otherwise z is 1; 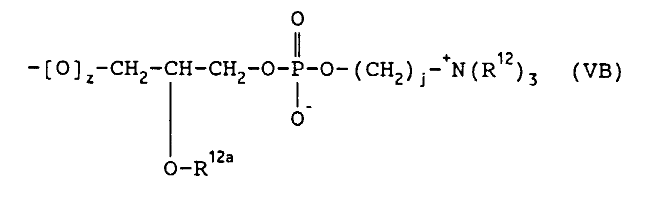 wherein the groups R¹² are the same or different and each is hydrogen, C₁-C₄ alkyl, aryl such as phenyl, or two of the groups R¹², together with the nitrogen atom to which they are attached, form a heterocyclic ring containing from 5 to 7 atoms or the three groups R¹², together with the nitrogen atom to which they are attached, form a fused ring structure containing from 5 to 7 atoms in each ring,
R^{12a} is a group of formula -C(O)R^{12b}, in which R^{12b} is a group containing an -NRC(O)NR- or -NRC(O)O- moiety capable of stably binding to a polyurethane substrate surface by physisorption;
j is from 1 to 20; and
if B is other than a valence bond, z is 1 and if B is a valence bond z is 0 if X is directly bonded to an oxygen or nitrogen atom and otherwise z is 1; 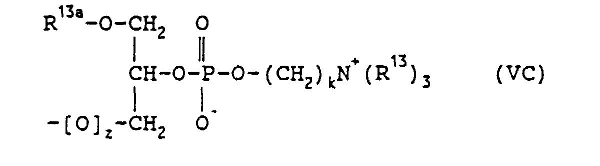 wherein the groups R¹³ are the same or different and each is hydrogen, C₁₋₄ alkyl, aryl, such as phenyl, or two of the groups R¹³, together with the nitrogen atom to which they are attached, form a heterocyclic ring containing from 5 to 7 atoms or the three groups R¹³, together with the nitrogen atom to which they are attached, form a fused ring structure containing from 5 to 7 atoms in each ring,
R^{13a} is a group of formula -C(O)R^{13b}, in which R^{13b} is a group containing an -NRC(O)NR- or -NRC(O)O- moiety capable of stably binding to a polyurethane substrate surface by physisorption;
k is from 1 to 20; and
if B is other than a valence bond, z is 1 and if B is a valence bond z is 0 if X is directly bonded to an oxygen or nitrogen atom and otherwise z is 1; or analogues of any of the groups VA, VB, or VC in which the quaternary ammonium group NR₃' is replaced by a phosphonium group -⁺PR'ᵣ(OR')₃₋ᵣ where r is from 0 to 3, or a sulphonium group -⁺SR'₂, where R' corresponds to R¹¹, R¹², or R¹³ or a group Het⁺, where Het is a nitrogen, phosphorus or sulphur-containing heterocycle, such as a pyridinium group.

23. A compound of the formula VII or VIII 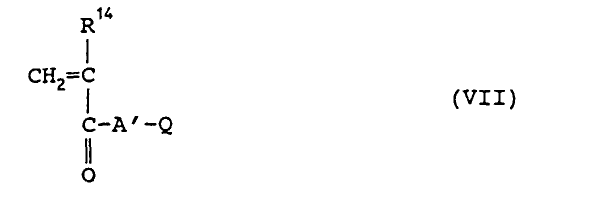 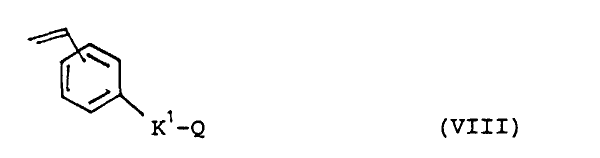 in which
R¹⁴ is hydrogen or C₁-C₄ alkyl,
A' is -O- or -NR¹⁵- where R¹⁵ is hydrogen or a C₁-C₄ alkyl group or R¹⁵ is a group Q;
K¹ is a group -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in which the groups R¹⁶ are the same or different), -(CH₂)ₗO-, -(CH₂)ₗSO₃-, a valence bond and 1 is from 1 to 12 and R¹⁶ is hydrogen or a C₁-C₄ alkyl group; and
Q is a group of formula (IXA) or (IXB)
-R¹⁷-NRC(O)NR-R¹⁸ (IXA)
-R¹⁷-NRC(O)O-R¹⁸ (IXB)
wherein R, (or the two groups R in (IXA), which may be the same or different each) is hydrogen or C₁₋₄ alkyl;
R¹⁷ is alkylene, arylene, alkylarylene, arylalkylene or alkylarylalkylene, cycloalkylene, alkylcycloalkylene, cycloalkyl-alkylene or alkyl-cycloalkyl-alkylene; and
R¹⁸ is alkyl, aryl or alkylaryl or cycloalkyl or alkylcycloalkyl wherein the alkyl groups may optionally be interrupted by one or more arylene or cycloalkylene groups, and wherein R¹⁸ may optionally contain one or more groups -NRC(O)NR- or -NRC(O)O- as hereinbefore defined.

24. A compound according to claim 23 selected from
phenylaminocarbonylaminophenylmethylphenylaminocarbonylaminoethyl methacrylate;
cyclohexylaminocarbonylaminocyclohexylmethylcyclohexylaminocarbonylaminoethyl methacrylate;
butyloxycarbonylaminoethyl methacrylate;
benzyloxycarbonylaminoethyl methacrylate;
phenyloxycarbonylaminoethyl methacrylate; and
cyclohexyloxycarbonylaminoethyl methacylate.

25. A process for producing a compound according to claim 23 which comprises reacting a corresponding compound containing, in place of the group of formula (IXA) or (IXB), a group of formula (X)
-R¹⁷-NCO (X)
where
R¹⁷ is as hereinbefore defined in claim 23 with
(a) a compound R¹⁸-NH₂ or an activated derivative thereof where R¹⁸ is as defined in claim 23 to produce a compound containing a group of formula (IXA); or
(b) a compound R¹⁸-OH, or an activated derivative thereof where R¹⁸ is as defined in claim 23 to produce a compound containing a group of formula (IXB).

## Patentansprüche

1. Polymer von einem oder mehreren radikalisch polymerisierbaren Monomeren, erhalten durch Copolymerisation eines radikalisch polymerisierbaren ethylenisch ungesättigten Monomers, das eine zwitterionische Gruppe enthält, und eines radikalisch polymerisierbaren ethylenisch ungesättigten Comonomers der allgemeinen Formel (VI)
Y¹-Q (VI)
worin
Y¹ eine aus 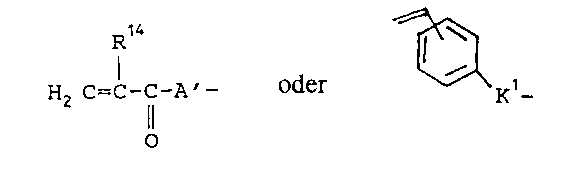 ausgewählte ethylenisch ungesättigte polymerisierbare Gruppe ist, wobei R¹⁴ für Wasserstoff oder C₁-C₄-Alkyl steht,
A' -O- oder -NR¹⁵- bedeutet, wobei R¹⁵ für Wasserstoff oder eine C₁-C₄-Alkylgruppe steht oder R¹⁵ eine Gruppe Q ist;
K¹ eine Gruppe -(CH₂)ₗOC(O)-, -(CH₂)ₗC(O)O-, -(CH₂)ₗOC(O)O-,
-(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in welcher die Gruppen R¹⁶ gleich oder verschieden sind), -(CH₂)ₗO-, -(CH₂)ₗSO₃-, eine Valenzbindung repräsentiert und 1 1 bis 12 ist und R¹⁶ Wasserstoff oder eine C₁-C₄-Alkylgruppe darstellt; und Q eine Gruppe der Formel (IXA) oder (IXB) ist
-R¹⁷-NRC(O)NR-R¹⁸ (IXA)
-R¹⁷-NRC(O)O-R¹⁸ (IXB)
worin R (oder die beiden Gruppen R in (IXA), die gleich oder verschieden sein können, jeweils) für Wasserstoff oder C₁-C₄-Alkyl steht;
R¹⁷ Alkylen, Arylen, Alkylarylen, Arylalkylen oder Alkylarylalkylen, Cycloalkylen, Alkylcycloalkylen, Cycloalkyl-alkylen oder Alkyl-cycloalkyl-alkylen ist; und
R¹⁸ Alkyl, Aryl oder Alkylaryl oder Cycloalkyl oder Alkylcycloalkyl darstellt, worin die Alkylgruppen gegebenenfalls durch eine oder mehrere Arylen- oder Cycloalkylengruppen unterbrochen sein können und wobei R¹⁸ gegebenenfalls eine oder mehrere Gruppen -NRC(O)NR- oder -NRC(O)O- wie oben definiert enthalten kann, wobei die Gruppe Q in der Lage ist, das Polymer durch Physisorption stabil an eine polymere Substratoberfläche zu binden, oder Kompatibilität mit einem Grundpolymer in einer Mischung bereitstellen kann.

2. Polymer nach Anspruch 1, umfassend Reste von einem oder mehreren Verdünnungs-Comonomeren und/oder reaktiven Comonomeren, um Stellen der Anbindung für einen Liganden oder Vernetzung bereitzustellen.

3. Polymer nach Anspruch 1 oder Anspruch 2, in welchem das Zentrum permanenter positiver Ladung in dem zwitterionischen Monomer durch ein quaternäres Stickstoffatom bereitgestellt wird und das Zentrum negativer Ladung durch eine Phosphatgruppe bereitgestellt wird.

4. Polymer nach irgendeinem vorangehenden Anspruch, in welchem das zwitterionische Monomer die allgemeine Formel (I)
Y-B-X (I)
aufweist, worin
B eine gerade oder verzweigte Alkylen-, Oxaalkylen- oder Oligooxaalkylen-Kette, die gegebenenfalls ein oder mehrere Fluoratome bis zu und einschließlich perfluorierter Ketten enthält, oder, falls X eine Kohlenstoff-Kohlenstoff-Kette zwischen B und der zwitterionischen Gruppe enthält oder falls Y ein an B gebundenes endständiges Kohlenstoffatom enthält, eine Valenzbindung ist;
X eine zwitterionische Gruppe ist und
Y eine ethylenisch ungesättigte polymerisierbare Gruppe ist, die ausgewählt ist aus 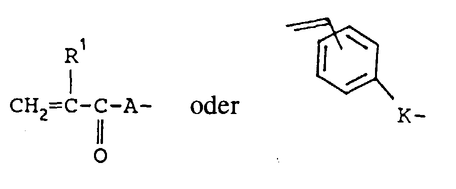 worin
R¹ für Wasserstoff oder eine C₁-C₄-Alkylgruppe steht;
A -O- oder -NR²- ist, wobei R² Wasserstoff oder eine C₁-C₄-Alkylgruppe repräsentiert oder R² -B-X bedeutet, wobei B und X wie oben definiert sind; und
K eine Gruppe -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³- (in welcher die Gruppen R³ gleich oder verschieden sind), -(CH₂)ₚO-, -(CH₂)ₚSO₃- oder, gegebenenfalls in Kombination mit B, eine Valenzbindung darstellt und p 1 bis 12 ist und R³ für Wasserstoff oder eine C₁-C₄-Alkylgruppe steht.

5. Polymer nach Anspruch 4, in welchem das Monomer die Formel 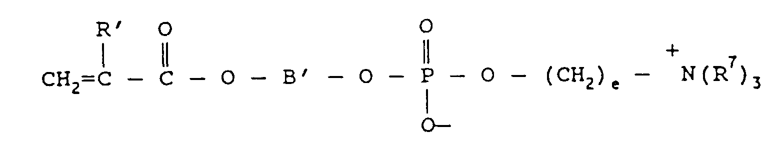 aufweist, worin R' für Wasserstoff oder Methyl steht, B' Alkylen mit bis zu 12 Kohlenstoffatomen repräsentiert, die Gruppen R⁷ gleich oder verschieden sind und jeweils Wasserstoff, C₁-C₄-Alkyl, Aryl wie beispielsweise Phenyl bedeuten oder zwei der Gruppen R⁷ zusammen mit dem Stickstoffatom, an das sie geknüpft sind, einen 5 bis 7 Atome enthaltenden heterocyclischen Ring bilden oder die drei Gruppen R⁷ zusammen mit dem Stickstoffatom, an das sie geknüpft sind, eine 5 bis 7 Atome in jedem Ring enthaltende kondensierte Ringstruktur bilden und e 1 bis 20 ist.

6. Polymer nach Anspruch 5, in welchem R⁷ jeweils für Wasserstoff oder C₁₋₄-Alkyl steht, vorzugsweise alle Methyl sind.

7. Polymer nach irgendeinem vorangehenden Anspruch, in welchem das Comonomer die Formel 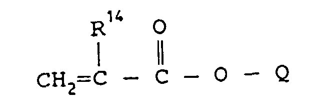 aufweist.

8. Polymer nach Anspruch 7, in welchem R für Wasserstoff, Methyl oder Ethyl, vorzugsweise Wasserstoff, steht.

9. Polymer nach irgendeinem vorangehenden Anspruch, umfassend Reste eines Comonomers, das eine reaktive Gruppe der allgemeinen Formel (XI) trägt
Y²-Q¹ (XI)
worin Y² eine ethylenisch ungesättigte polymerisierbare Gruppe darstellt, die ausgewählt ist aus 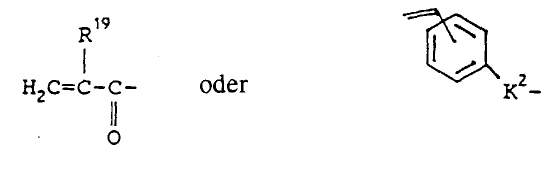 worin
R¹⁹ Wasserstoff oder C₁-C₄-Alkyl ist,
K² eine Gruppe -(CH₂)_{q}OC(O)-, -(CH₂)_{q}C(O)O-, -(CH₂)_{q}OC(O)O-, -(CH₂)_{q}NR²⁰-, -(CH₂)_{q}NR²⁰C(O)-, -(CH₂)_{q}C(O)NR²⁰-, -(CH₂)_{q}NR²⁰C(O)O-, -(CH₂)_{q}OC(O)NR²⁰-, -(CH₂)_{q}NR²⁰C(O)NR²⁰- (in welcher die Gruppen R²⁰ gleich oder verschieden sind), -(CH₂)_{q}O- oder -(CH₂)_{q}SO₃- oder eine Valenzbindung darstellt und q 1 bis 20 ist und R²⁰ für Wasserstoff oder eine C₁-C₄-Alkylgruppe steht; und
Q¹ eine reaktive Gruppe darstellt, die in der Lage ist, unter Bereitstellung von kovalenter Bindung oder Vernetzung zu reagieren, ausgewählt aus Gruppen, die Cinnamyl-, Epoxy-, -CHOHCH₂Hal (worin Hal ein Halogenatom darstellt), Methylol- oder Silylgruppe enthalten, einer ethylenisch ungesättigten vernetzbaren Gruppe wie beispielsweise einer acetylenischen, diacetylenischen, vinylischen oder divinylischen Gruppe, einer Acetoacetoxygruppe oder Chloralkylsulfongruppe, vorzugsweise Chlorethylsulfongruppe, oder einer Hydroxyl-, Amino- oder Carbonsäuregruppe oder einem aktivierten Derivat derselben wie beispielsweise einer Succinimido-, Tosylat-, Triflat-, Imidazolcarbonylamino- oder substituierten Triazingruppe.

10. Verfahren zur Herstellung eines Polymers nach Anspruch 1 durch Copolymerisation des Monomers, das die zwitterionische Gruppe enthält, und des Comonomers, das die Gruppe Q, die eine Polyurethan-ähnliche Gruppe einschließt, enthält, gegebenenfalls mit einem Verdünnungs- und/oder reaktiven Comonomer.

11. Verfahren nach Anspruch 10, umfassend 5 bis 75 Mol-% Reste eines Verdünnungs-Monomers und/oder 0,1 bis 20 Mol-% eines reaktiven Comonomers.

12. Produkt, umfassend ein mit einem Polymer nach irgendeinem der Ansprüche 1 bis 9 beschichtetes polymeres Substrat oder ein damit gemischtes Grundpolymer.

13. Produkt nach Anspruch 12, in welchem das polymere Substrat oder Grundpolymer ein Polyurethan ist.

14. Produkt nach Anspruch 12 oder 13, bei dem es sich um eine Vorrichtung handelt, die mit Blut oder Protein oder Mikroorganismen in Berührung gebracht werden soll.

15. Verfahren zur Biokompatibilisierung eines polymeren Substrats durch Beschichtung der Oberfläche desselben mit einem Polymer nach irgendeinem der Ansprüche 1 bis 9.

16. Verfahren nach Anspruch 15, in welchem das polymere Substrat ein Polyurethan umfaßt.

17. Verfahren nach Anspruch 15 oder 16, in welchem die Blutplättchen-Aktivierung der Oberfläche des Substrats durch den Überzug um mindestens 70% vermindert wird.

18. Verfahren nach irgendeinem der Ansprüche 15 bis 17, in welchem die Fibrinogen-Absorption der Oberfläche des Substrats durch den Überzug um mindestens 60% vermindert wird.

19. Verfahren, in welchem ein Grundpolymer und ein Polymer nach irgendeinem der Ansprüche 1 bis 9 zusammengemischt werden.

20. Verfahren nach Anspruch 19, in welchem das Grundpolymer ein Polyurethan ist.

21. Verfahren nach Anspruch 19 oder 20, in welchem die Mischung unter Bildung eines Gegenstandes geformt wird.

22. Verbindung der Formel II oder III 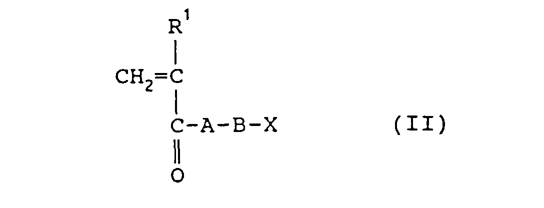 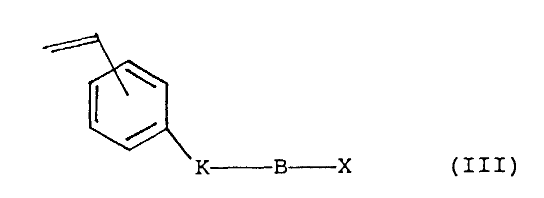 worin
B eine gerade oder verzweigte Alkylen-, Oxaalkylen- oder Oligooxaalkylen-Kette, die gegebenenfalls ein oder mehrere Fluoratome bis zu und einschließlich perfluorierter Ketten enthält, oder, falls X eine Kohlenstoff-Kohlenstoff-Kette zwischen B und der zwitterionischen Gruppe enthält oder falls Y ein an B gebundenes endständiges Kohlenstoffatom enthält, eine Valenzbindung ist;
R¹ für Wasserstoff oder eine C₁-C₄-Alkylgruppe steht;
A -O- oder -NR²- darstellt, wobei R² Wasserstoff oder eine C₁-C₄-Alkylgruppe repräsentiert oder R² für -B-X steht, wobei B wie oben definiert ist; und
K eine Gruppe -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³- (in welcher die Gruppen R³ gleich oder verschieden sind), -(CH₂)ₚO-, -(CH₂)ₚSO₃- oder, gegebenenfalls in Kombination mit B, eine Valenzbindung darstellt und p 1 bis 12 ist und R³ für Wasserstoff oder eine C₁-C₄-Alkylgruppe steht und
X ausgewählt ist aus Gruppen VA, VB und VC; 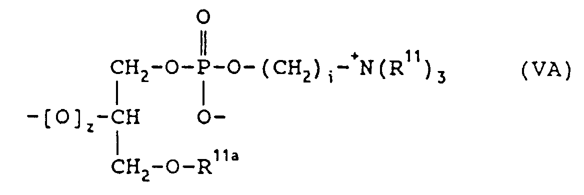 worin die Gruppen R¹¹ gleich oder verschieden sind und jeweils für Wasserstoff, C₁₄-Alkyl, Aryl wie beispielsweise Phenyl stehen oder zwei der Gruppen R¹¹ zusammen mit dem Stickstoffatom, an das sie geknüpft sind, einen 5 bis 7 Atome enthaltenden heterocyclischen Ring bilden oder drei Gruppen R¹¹ zusammen mit dem Stickstoffatom, an das sie geknüpft sind, eine 5 bis 7 Atome in jedem Ring enthaltende kondensierte Ringstruktur bilden,
R^{11a} eine Gruppe der Formel -C(O)R^{11b} ist, in welcher R^{11b} eine eine -NRC(O)NR- oder -NRC(O)O-Einheit enthaltende Gruppe, die zur stabilen Bindung an eine polymere Substratoberfläche durch Physisorption in der Lage ist, bedeutet,
i 1 bis 20 ist; und,
falls B von einer Valenzbindung verschieden ist, z gleich 1 ist und, falls B eine Valenzbindung ist, z gleich 0 ist, wenn X direkt an ein Sauerstoff- oder Stickstoffatom gebunden ist, und ansonsten z gleich 1 ist; 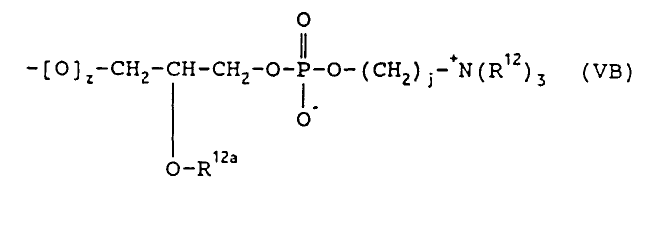 worin die Gruppen R¹² gleich oder verschieden sind und jeweils für Wasserstoff, C₁-C₄-Alkyl, Aryl wie beispielsweise Phenyl stehen oder zwei der Gruppen R¹² zusammen mit dem Stickstoffatom, an das sie geknüpft sind, einen 5 bis 7 Atome enthaltenden heterocyclischen Ring bilden oder die drei Gruppen R¹² zusammen mit dem Stickstoffatom, an das sie geknüpft sind, eine 5 bis 7 Atome in jedem Ring enthaltende kondensierte Ringstruktur bilden,
R^{12a} eine Gruppe der Formel -C(O)R^{12b} ist, worin R^{12b} eine Gruppe darstellt, die eine -NRC(O)NR- oder -NRC(O)O-Einheit, die zur stabilen Bindung an eine Polyurethan-Substratoberfläche durch Physisorption in der Lage ist, enthält,
j 1 bis 20 ist; und,
falls B von einer Valenzbindung verschieden ist, z gleich 1 ist und, falls B eine Valenzbindung ist, z gleich 0 ist, wenn X direkt an ein Sauerstoff- oder Stickstoffatom gebunden ist, und ansonsten z gleich 1 ist; 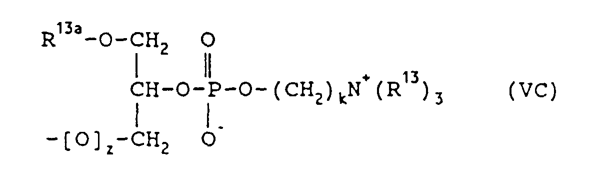 worin die Gruppen R¹³ gleich oder verschieden sind und jeweils für Wasserstoff, C₁-C₄-Alkyl, Aryl wie beispielsweise Phenyl stehen oder zwei der Gruppen R¹³ zusammen mit dem Stickstoffatom, an das sie geknüpft sind, einen 5 bis 7 Atome enthaltenden heterocyclischen Ring bilden oder die drei Gruppen R¹³ zusammen mit dem Stickstoffatom, an das sie geknüpft sind, eine 5 bis 7 Atome in jedem Ring enthaltende kondensierte Ringstruktur bilden,
R^{13a} eine Gruppe der Formel -C(O)R^{13b} ist, worin R^{13b} eine Gruppe darstellt, die eine -NRC(O)NR- oder -NRC(O)O-Einheit, die zur stabilen Bindung an eine Polyurethan-Substratoberfläche durch Physisorption in der Lage ist, enthält,
k 1 bis 20 ist; und,
falls B von einer Valenzbindung verschieden ist, z gleich 1 ist und, falls B eine Valenzbindung ist, z gleich 0 ist, wenn X direkt an ein Sauerstoff- oder Stickstoffatom gebunden ist, und ansonsten z gleich 1 ist; oder Analoga irgendeiner der Gruppen VA, VB oder VC, in denen die quaternäre Ammoniumgruppe NR₃' durch eine Phosphoniumgruppe -⁺PR'ᵣ(OR')_{3-r,}, worin r 0 bis 3 ist, oder eine Sulfoniumgruppe -⁺SR'₂, wobei R¹¹, R¹² oder R¹³ entspricht, oder eine Gruppe Het⁺, worin Het ein Stickstoff-, Phosphor- oder Schwefel-haltiger Heterozyklus ist, wie beispielsweise eine Pyridiniumgruppe, ersetzt ist.

23. Verbindung der Formel VII oder VIII 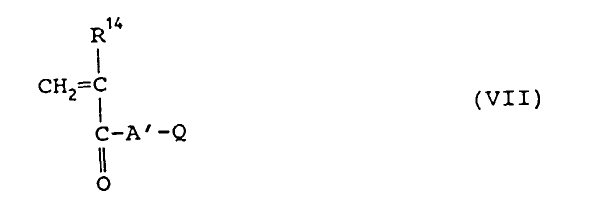 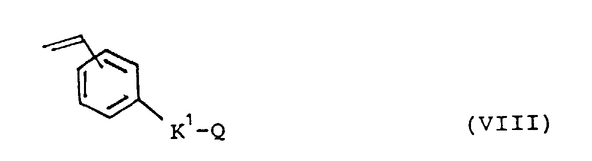 in welcher
R¹⁴ für Wasserstoff oder C₁-C₄-Alkyl steht,
A' -O- oder -NR¹⁵- ist, wobei R¹⁵ Wasserstoff oder eine C₁-C₄-Alkylgruppe darstellt oder R¹⁵ eine Gruppe Q ist;
K¹ eine Gruppe -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (in welcher die Gruppen R¹⁶ gleich oder verschieden sind), -(CH₂)ₗO-, -(CH₂)ₗSO₃-, eine Valenzbindung repräsentiert und l 1 bis 12 ist und R¹⁶ Wasserstoff oder eine C₁-C₄-Alkylgruppe darstellt; und
Q eine Gruppe der Formel (IXA) oder (IXB) ist
-R¹⁷-NRC(O)NR-R¹⁸ (IXA)
-R¹⁷-NRC(O)O-R¹⁸ (IXB)
worin R (oder die beiden Gruppen R in (IXA), die gleich oder verschieden sein können, jeweils) für Wasserstoff oder C₁-C₄-Alkyl steht;
R¹⁷ Alkylen, Arylen, Alkylarylen, Arylalkylen oder Alkylarylalkylen, Cycloalkylen, Alkylcycloalkylen, Cycloalkyl-alkylen oder Alkyl-cycloalkyl-alkylen ist; und
R¹⁸ Alkyl, Aryl oder Alkylaryl oder Cycloalkyl oder Alkylcycloalkyl darstellt, worin die Alkylgruppen gegebenenfalls durch eine oder mehrere Arylen- oder Cycloalkylengruppen unterbrochen sein können und wobei R¹⁸ gegebenenfalls eine oder mehrere Gruppen -NRC(O)NR- oder -NRC(O)O- wie oben definiert enthalten kann.

24. Verbindung nach Anspruch 23, ausgewählt aus
Phenylaminocarbonylaminophenylmethylphenylaminocarbonylaminoethylmethacrylat;
Cyclohexylaminocarbonylaminocyclohexylmethylcyclohexylaminocarbonylaminoethylmethacrylat;
Butyloxycarbonylaminoethylmethacrylat;
Benzyloxycarbonylaminoethylmethacrylat;
Phenyloxycarbonylaminoethylmethacrylat; und
Cyclohexyloxycarbonylaminoethylmethacrylat.

25. Verfahren zur Herstellung einer Verbindung nach Anspruch 23, umfassend die Umsetzung einer entsprechenden Verbindung, die anstelle der Gruppe der Formel (IXA) oder (IXB) eine Gruppe der Formel (X) enthält
-R¹⁷-NCO (X)
worin R¹⁷ wie oben in Anspruch 23 definiert ist, mit
(a) einer Verbindung R¹⁸-NH₂ oder einem aktivierten Derivat derselben, worin R¹⁸ wie in Anspruch 23 definiert ist, um eine eine Gruppe der
Formel (IXA) enthaltende Verbindung herzustellen; oder (b) einer Verbindung R¹⁸-OH oder einem aktivierten Derivat derselben, worin R¹⁸ wie in Anspruch 23 definiert ist, um eine eine Gruppe der Formel (IXB) enthaltende Verbindung herzustellen.

## Revendications

1. Polymère d'un ou plusieurs monomères polymérisables par voie radicalaire, obtenu par copolymérisation d'un monomère éthyléniquement insaturé polymérisable par voie radicalaire contenant un groupement zwitterionique et d'un comonomère éthyléniquement insaturé polymérisable par voie radicalaire de formule générale (VI) :
Y¹-Q (VI)
dans laquelle Y¹ représente un groupement polymérisable éthyléniquement insaturé choisi parmi les suivants : 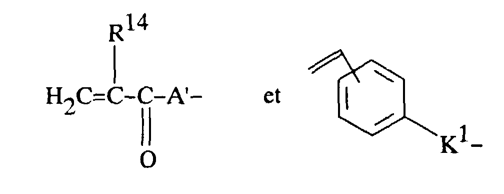 où
R¹⁴ représente un atome d'hydrogène ou un groupement alkyle en C₁-C₄,
A' représente -O- ou -NR¹⁵-, R¹⁵ étant un atome d'hydrogène ou un groupement alkyle en C₁-C₄ ou R¹⁵ étant un groupement Q ;
K¹ représente un groupement -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (où les groupements R¹⁶ sont identiques ou différents), -(CH₂)ₗO-, -(CH₂)ₗSO₃-, une liaison de valence, et l est compris entre 1 et 12 et R¹⁶ représente un atome d'hydrogène ou un groupement alkyle en C₁-C₄; et
Q représente un groupement de formule (IXA) ou (IXB) :
-R¹⁷-NRC(O)NR-R¹⁸ (IXA)
-R¹⁷-NRC(O)O-R¹⁸ (IXB)
où
R (ou les deux groupements R dans (IXA), qui peuvent être identiques ou différents) représente un atome hydrogène ou un groupement alkyle en C₁-C₄;
R¹⁷ représente un groupement du type alkylène, arylène, alkylarylène, arylalkylène ou alkylarylalkylène, cycloalkylène, alkylcycloalkylène, cycloalkylalkylène ou alkylcycloalkylalkylène ; et
R¹⁸ représente un groupement du type alkyle, aryle ou alkylaryle ou cycloalkyle ou alkylcycloalkyle, où les groupements alkyle peuvent être éventuellement interrompus par un ou plusieurs groupements arylène ou cycloalkylène et où R¹⁸ peut éventuellement contenir un ou plusieurs groupements -NRC(O)NR- ou -NRC(O)O- tels que définis précédemment, ledit groupement Q étant capable de lier de façon stable le polymère par physisorption à une surface de substrat polymère ou capable de fournir une compatibilité avec un polymère de base dans un mélange.

2. Polymère selon la revendication 1, comprenant les résidus d'un ou plusieurs comonomères diluants et/ou comonomères réactifs pour fournir des points de fixation pour un ligand ou la réticulation.

3. Polymère selon la revendication 1 ou 2, dans lequel le centre de charge positive permanente du monomère zwitterionique est fourni par un atome d'azote quaternaire et le centre de charge négative est fourni par un groupement phosphate.

4. Polymère selon l'une quelconque des revendications précédentes, dans lequel le monomère zwitterionique possède la formule générale (I) :
Y-B-X (I)
dans laquelle :
B est une chaîne linéaire ou ramifiée du type alkylène, oxalkylène ou oligo-oxalkylène contenant éventuellement un ou plusieurs atomes de fluor et incluant des chaînes perfluorées ou, lorsque X contient une chaîne carbone-carbone entre B et le groupement zwitterionique ou que Y contient un atome de carbone terminal lié à B, une liaison de valence ;
X représente un groupement zwitterionique ; et
Y représente un groupement polymérisable éthyléniquement insaturé choisi parmi : 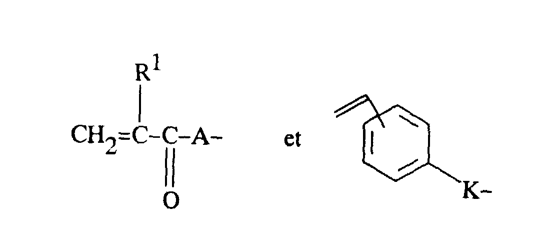 dans lesquelles :
R¹ représente un atome d'hydrogène ou un groupement alkyle en C₁-C₄ ;
A représente -O- ou -NR²-, R² représentant un atome d'hydrogène ou un groupement alkyle en C₁-C₄ ou R² représentant -B-X, où B et X sont tels que définis ci-dessus ; et
K représente un groupement -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³- (où les groupements R³ sont identiques ou différents), -(CH₂)ₚO-, -(CH₂)ₚSO₃-, ou, éventuellement en association avec B, une liaison de valence, et p est compris entre 1 et 12 et R³ représente un atome d'hydrogène ou un groupement alkyle en C₁-C₄.

5. Polymère selon la revendication 4, dans lequel le monomère a la formule: 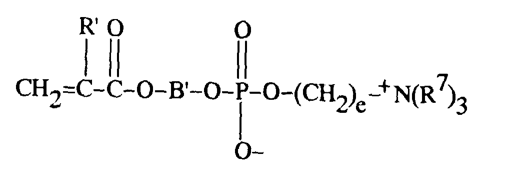 dans laquelle R' est un hydrogène ou un méthyle, B' est un alkylène ayant jusqu'à 12 atomes de carbone, les groupements R⁷ sont identiques ou différents et représentent chacun un radical du type hydrogène, alkyle en C₁-C₄, aryle, tel que phényle, ou deux des groupements R⁷, avec l'atome d'azote auquel ils sont liés, forment un noyau hétérocyclique contenant de 5 à 7 atomes ou les trois groupements R⁷, avec l'atome d'azote auquel ils sont liés, forment une structure de noyaux condensés contenant de 5 à 7 atomes dans chaque noyau et e est compris entre 1 et 20.

6. Polymère selon la revendication 5, dans lequel les radicaux R⁷ représentent chacun un atome d'hydrogène ou un groupement alkyle en C₁-C₄, de préférence tous des méthyles.

7. Polymère selon l'une quelconque des revendications précédentes, dans lequel ledit comonomère présente la formule :

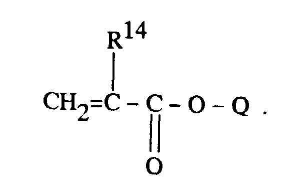

8. Polymère selon la revendication 7, dans lequel R représente un hydrogène, un méthyle ou un éthyle, de préférence un hydrogène.

9. Polymère selon l'une quelconque des revendications précédentes, comprenant les résidus d'un comonomère portant un groupement réactif de formule générale (XI) :
Y²-Q¹ (XI)
dans laquelle :
Y² représente un groupement polymérisable éthyléniquement insaturé choisi parmi : 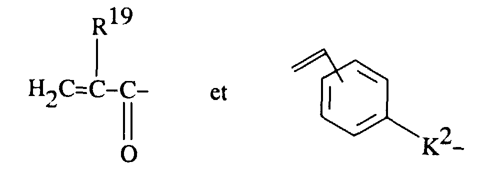 où
R¹⁹ représente un hydrogène ou un groupement alkyle en C₁-C₄,
K² représente un groupement -(CH₂)_{q}OC(O)-, -(CH₂)_{q}C(O)O-, -(CH₂)_{q}OC(O)O-, -(CH₂)_{q}NR²⁰-, -(CH₂)_{q}NR²⁰C(O)-, -(CH₂)_{q}C(O)NR²⁰-, -(CH₂)_{q}NR²⁰C(O)O-, -(CH₂)_{q}OC(O)NR²⁰-, -(CH₂)_{q}NR²⁰C(O)NR²⁰- (où les groupements R²⁰ sont identiques ou différents), -(CH₂)_{q}O- ou -(CH₂)_{q}SO₃-, ou bien une liaison de valence, et q est compris entre 1 et 12 et R²⁰ représente un atome d'hydrogène ou un groupement alkyle en C₁-C₄ ; et
Q¹ représente un groupement réactif capable d'une réaction pour fournir une liaison covalente, ou d'une réticulation, choisi parmi les groupements contenant un groupement cinnamyle, époxy, -CHOHCH₂Hal (Hal représentant un atome d'halogène), méthylol ou silyle, un groupement réticulable éthyléniquement insaturé tel qu'un groupement acétylénique, diacétylénique, vinylique ou divinylique, un groupement acétoacétoxy ou un groupement chloroalkylsulfone, de préférence chloroéthylsulfone, ou un groupement hydroxyle, amino ou acide carboxylique ou un de leurs dérivés activés, tels qu'un groupement succinimido, tosylate, triflate, imidazole carbonylamino ou triazine substituée.

10. Procédé de préparation d'un polymère selon la revendication 1, par copolymérisation dudit monomère contenant un groupement zwitterionique et dudit comonomère contenant le groupement Q qui inclut un groupement de type polyuréthane, éventuellement avec un comonomère diluant et/ou réactif.

11. Procédé selon la revendication 10, comprenant de 5 à 75 % en mole de résidus d'un monomère diluant et/ou de 0,1 à 20 % en mole de résidus d'un comonomère réactif.

12. Produit comprenant un substrat polymère revêtu d'un polymère selon l'une quelconque des revendications 1 à 9 ou un polymère de base mélangé avec un polymère selon l'une quelconque des revendications 1 à 9.

13. Produit selon la revendication 12, dans lequel le substrat polymère ou le polymère de base est un polyuréthane.

14. Produit selon la revendication 12 ou 13, qui est un dispositif à mettre en contact avec du sang ou une protéine ou des micro-organismes.

15. Procédé consistant à rendre biocompatible un substrat polymère par revêtement de sa surface avec un polymère selon l'une quelconque des revendications 1 à 9.

16. Procédé selon la revendication 15, dans lequel le substrat polymère comprend un polyuréthane.

17. Procédé selon la revendication 15 ou 16, dans lequel l'activation plaquettaire à la surface du substrat est réduite d'au moins 70 % par le revêtement.

18. Procédé selon l'une quelconque des revendications 15 à 17, dans lequel l'absorption du fibrinogène à la surface du substrat est réduite d'au moins 60 % par le revêtement.

19. Procédé dans lequel un polymère de base et un polymère selon l'une quelconque des revendications 1 à 9 sont mélangés.

20. Procédé selon la revendication 19, dans lequel le polymère de base est un polyuréthane.

21. Procédé selon la revendication 19 ou 20, dans lequel le mélange est façonné pour former un article.

22. Composé de formule (II) ou (III) : 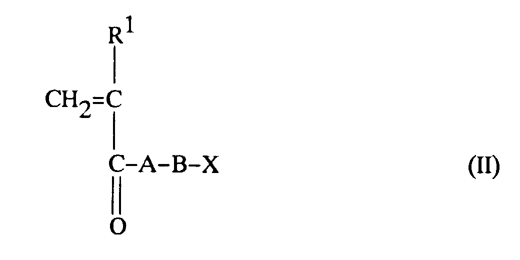 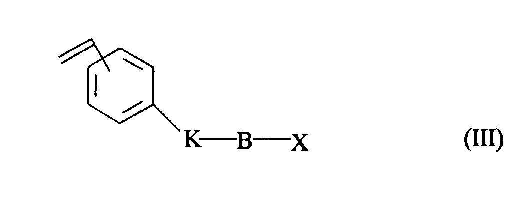 dans lesquelles :
B représente une chaîne linéaire ou ramifiée de type alkylène, oxalkylène ou oligo-oxalkylène contenant éventuellement un ou plusieurs atomes de fluor et comprenant des chaînes perfluorées ou, lorsque X contient une chaîne carbone-carbone entre B et le groupement zwitterionique ou que Y contient un atome de carbone terminal lié à B, une liaison de valence ;
R¹ représente un atome d'hydrogène ou un groupement alkyle en C₁-C₄ ;
A représente -O- ou -NR²-, R² représentant un atome d'hydrogène ou un groupement alkyle en C₁-C₄ ou R² représentant -B-X, où B est tel que défini ci-dessus ; et
K représente un groupement -(CH₂)ₚOC(O)-, -(CH₂)ₚC(O)O-, -(CH₂)ₚOC(O)O-, -(CH₂)ₚNR³-, -(CH₂)ₚNR³C(O)-, -(CH₂)ₚC(O)NR³-, -(CH₂)ₚNR³C(O)O-, -(CH₂)ₚOC(O)NR³-, -(CH₂)ₚNR³C(O)NR³- (où les groupements R³ sont identiques ou différents), -(CH₂)ₚO-, -(CH₂)ₚSO₃-, ou, éventuellement en association avec B, une liaison de valence, et p est compris entre 1 et 12 et R³ représente un atome d'hydrogène ou un groupement alkyle en C₁-C₄; et
X est choisi parmi les groupements VA, VB et VC : 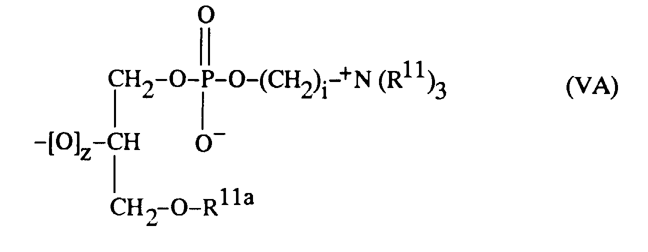
où
les groupements R¹¹ sont identiques ou différents et représentent chacun un radical du type hydrogène, alkyle en C₁-C₄, aryle, tel que phényle, ou deux des groupements R¹¹, avec l'atome d'azote auquel ils sont liés, forment un noyau hétérocyclique contenant de 5 à 7 atomes ou les trois groupements R¹¹, avec l'atome d'azote auquel ils sont liés, forment une structure de noyaux condensés contenant de 5 à 7 atomes dans chaque noyau,
R^{11a} représente un groupement de formule -C(O)R^{11b}, dans laquelle R^{11b} est un groupement contenant une fraction -NRC(O)NR- ou -NRC(O)O- capable d'une liaison stable à une surface de substrat polymère par physisorption,
i est compris entre 1 et 20 ; et
lorsque B n'est pas une liaison de valence, z vaut 1 et lorsque B est une liaison de valence, z vaut 0 lorsque X est directement lié à un atome d'oxygène ou d'azote, et sinon z vaut 1 ; 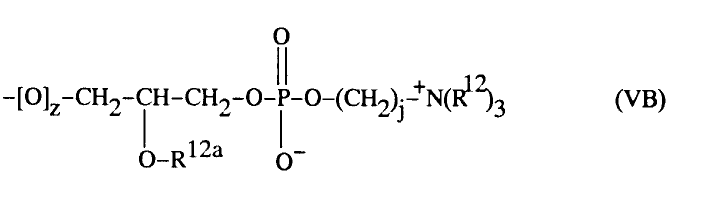 où
les groupements R¹² sont identiques ou différents et représentent chacun un radical du type hydrogène, alkyle en C₁-C₄, aryle, tel que phényle, ou deux des groupements R¹², avec l'atome d'azote auquel ils sont liés, forment un noyau hétérocyclique contenant de 5 à 7 atomes ou les trois groupements R¹², avec l'atome d'azote auquel ils sont liés, forment une structure de noyaux condensés contenant de 5 à 7 atomes dans chaque noyau,
R^{12a} représente un groupement -C(O)R^{12b}, dans laquelle R^{12b} est un groupement contenant une fraction -NRC(O)NR- ou -NRC(O)O- capable d'une liaison stable à une surface de substrat de polyuréthane par physisorption,
j est compris entre 1 et 20 ; et
lorsque B n'est pas une liaison de valence, z vaut 1 et lorsque B est une liaison de valence, z vaut 0 lorsque X est directement lié à un atome d'oxygène ou d'azote, et sinon z vaut 1 ; 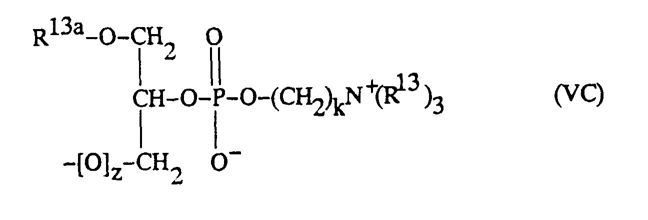 où
les groupements R¹³ sont identiques ou différents et représentent chacun un radical du type hydrogène, alkyle en C₁-C₄, aryle, tel que phényle, ou deux des groupements R¹³, avec l'atome d'azote auquel ils sont liés, forment un noyau hétérocyclique contenant de 5 à 7 atomes ou les trois groupements R¹³, avec l'atome d'azote auquel ils sont liés, forment une structure de noyaux condensés contenant de 5 à 7 atomes dans chaque noyau,
R^{13a} est un groupement de formule -C(O)R^{13b}, dans laquelle R^{13b} est un groupement contenant une fraction -NRC(O)NR- ou -NRC(O)O- capable d'une liaison stable à une surface de substrat de polyuréthane par physisorption,
k est compris entre 1 et 20 ; et
lorsque B n'est pas une liaison de valence, z vaut 1 et lorsque B est une liaison de valence, z vaut 0 lorsque X est directement lié à un atome d'oxygène ou d'azote, et sinon z vaut 1;
ou des analogues de l'un quelconque des groupements VA, VB ou VC dans lesquels le groupement ammonium quaternaire NR₃ est remplacé par un groupement phosphonium -⁺PR'ᵣ(OR')₃-ᵣ où r est compris entre 0 et 3, ou bien un groupement sulfonium -⁺SR'₂, où R' correspond à R¹¹, R¹² ou R¹³, ou un groupement Het⁺, où Het représente un hétérocycle contenant un azote, un phosphore ou un soufre, tel qu'un groupement pyridinium.

23. Composé de formule VII ou VIII : 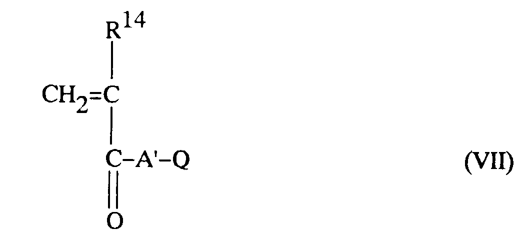 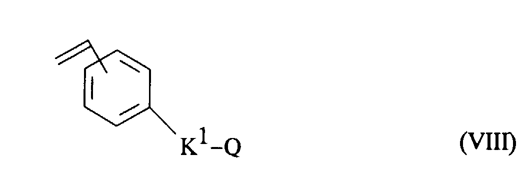 dans lesquelles :
R¹⁴ représente un atome d'hydrogène ou un groupement alkyle en C₁-C₄,
A' représente -O- ou -NR¹⁵-, R¹⁵ étant un atome d'hydrogène ou un groupement alkyle en C₁-C₄, ou R¹⁵ étant un groupement Q ;
K¹ représente un groupement -(CH₂)ₗOC(O)-, -(CH₂)ₗC(O)O-, -(CH₂)ₗOC(O)O-, -(CH₂)ₗNR¹⁶-, -(CH₂)ₗNR¹⁶C(O)-, -(CH₂)ₗC(O)NR¹⁶-, -(CH₂)ₗNR¹⁶C(O)O-, -(CH₂)ₗOC(O)NR¹⁶-, -(CH₂)ₗNR¹⁶C(O)NR¹⁶- (où les groupements R¹⁶ sont identiques ou différents), -(CH₂)ₗO-, -(CH₂)ₗSO₃-, une liaison de valence, et l est compris entre 1 et 12 et R¹⁶ représente un atome d'hydrogène ou un groupement alkyle en C₁-C₄ ; et
Q représente un groupement de formule (IXA) ou (IXB) :
-R¹⁷-NRC(O)NR-R¹⁸ (IXA)
-R¹⁷-NRC(O)O-R¹⁸ (IXB)
où
R (ou les deux groupements R dans (IXA), qui peuvent être identiques ou différents) représente un atome hydrogène ou un groupement alkyle en C₁-C₄ ;
R¹⁷ représente un groupement du type alkylène, arylène, alkylarylène, arylalkylène ou alkylarylalkylène, cycloalkylène, alkylcycloalkylène, cycloalkylalkylène ou alkylcycloalkylalkylène ; et
R¹⁸ représente un groupement du type alkyle, aryle ou alkylaryle ou cycloalkyle ou alkylcycloalkyle, où les groupements alkyle peuvent être éventuellement interrompus par un ou plusieurs groupements arylène ou cycloalkylène et où R¹⁸ peut éventuellement contenir un ou plusieurs groupements -NRC(O)NR- ou -NRC(O)O- tels que définis précédemment.

24. Composé selon la revendication 23, choisi parmi les suivants :
- méthacrylate de phénylaminocarbonylaminophénylméthylphénylaminocarbonylaminoéthyle ;
- méthacrylate de cyclohexylaminocarbonylaminocyclohexylméthylcyclohexylaminocarbonylaminoéthyle ;
- méthacrylate de butyloxycarbonylaminoéthyle;
- méthacrylate de benzyloxycarbonylaminoéthyle;
- méthacrylate de phényloxycarbonylaminoéthyle ; et
- méthacrylate de cyclohexyloxycarbonylaminoéthyle.

25. Un procédé de production d'un composé selon la revendication 23, qui comprend la réaction d'un composé correspondant contenant, à la place du groupement de formule (IXA) ou (IXB), un groupement de formule (X) :
-R¹⁷-NCO (X)
dans laquelle R¹⁷ est tel que défini précédemment dans la revendication 23, avec :
(a) un composé R¹⁸-NH₂ ou un de ses dérivés activés, où R¹⁸ est tel que défini dans la revendication 23, pour produire un composé contenant un groupement de formule (IXA) ; ou
(b) un composé R¹⁸-OH ou un de ses dérivés activés, où R¹⁸ est tel que défini dans la revendication 23, pour produire un composé contenant un groupement de formule (IXB).
